# EUROPEAN PATENT SPECIFICATION

(11) **EP 2 649 036 B1**
(45) Date of publication and mention of the grant of the patent: **09.09.2020**
(21) Application number: 12707448.2
(22) Date of filing: 22.02.2012
(51) Int. Cl.: C07C 67/03, C07C 69/82, C08J 11/24, C08K 5/00, C08L 67/02

(54) **FLAME RETARDANT POLY(BUTYLENE TEREPHTHALATE) ESTER COMPOSITIONS, METHODS OF MANUFACTURE, AND ARTICLES THEREOF**
FEUERFESTE POLY(BUTYLENTEREPHTHALAT-)ESTERZUSAMMENSETZUNGEN, HERSTELLUNGSVERFAHREN DAFÜR UND ARTIKEL DARAUS
COMPOSITIONS D'ESTER DE POLY(BUTYLENE TEREPHTHALATE) RETARDATRICES DE FLAMME, PROCÉDÉ DE PRÉPARATION ET LEURS ARTICLES

(30) Priority: 22.02.2011 US 201113032091
(43) Date of publication of application: 16.10.2013
(73) Proprietor: SABIC Global Technologies B.V., 4612 PX Bergen op Zoom (NL)
(72) Inventor: BELL, Philip Wesley, Mount Vernon, Indiana 47620 (US); SHAH, Dhaval, Evansville, Indiana 47712 (US)
(74) Representative: Balder IP Law, S.L.
(86) International application number: PCT/US2012/026046
(87) International publication number: WO 2012/116024

(56) References cited:
- EP-A1- 1 344 765
- WO-A1-95/11218
- WO-A1-2007/089598
- US-A- 5 391 263
- US-A1- 2002 111 409
- US-A1- 2010 168 336

## Description

### BACKGROUND

This disclosure relates to flame retardant poly(butylene terephthalate) compositions, methods of manufacture, and articles thereof, wherein the poly(butylene terephthalate) is manufactured in part from recycled or scrap polyesters.

Thermoplastic polyesters such as poly(butylene terephthalate) (PBT) are readily molded into useful articles, and articles comprising PBT have valuable characteristics including strength, toughness, high gloss, and solvent resistance. PBT therefore has utility in a wide range of applications, including automotive parts, electric appliances, and electronic devices. PBT is especially useful when blended with other components such as impact modifiers and polycarbonates.

Different prior art documents disclose a composition comprising a poly (alkylene terephthalate) and their use or their method of manufacturing.

The patent application WO2007/089598A1 describes a composition comprising a modified polybutylene terephthalate random copolymer that is derived from polyethylene terephthalate component and has at least one residue derived from the polyethylene terephthalate component, and at least a filler component.

Moreover, US2002/111409A1 describes a polyester resin compositions, including flame retardant compositions, having improved color stability. This composition contains a mixture of polyester resin, a quencher concentrate and an antimony compound as a synergist and/or a light-colored pigment.

US2010/168336A1 describes a process comprises depolymerizing, with 1,4-butane diol, a first polymer comprising a polyethylene terephthalate component in the presence of at least one second polymer to produce a molten mixture; and polymerizing the molten mixture under conditions sufficient to form a modified polybutylene terephthalate copolymer. The modified PBT comprises at least one polyethylene terephthalate component residue, and a member selected from the group consisting of (i) the at least one second polymer; (ii) the at least one residue derived from the second polymer; and (iii) combinations thereof.

The present invention also comprises a dimethyl terephthalate residual composition comprising a dimethyl terephthalate. Different prior art documents disclose dimethyl terephthalate compositions.

EP1344765 A1 describes a dimethyl terephthalate composition includes methyl 4-(1,3-dioxolan-2-yl)benzoate and dimethyl hydroxyterephthalate contained in dimethyl terephthalate, and exhibits improved properties as a material for producing polyester.

WO 95/11218A1 describes a method for separating and removing impurities from their mixture with dimethyl terephthalate (DMT), formed during a depolymerization process.

US5391263A1 describes the separation of ethylene glycol and diethylene glycol from dimethyl terephthalate is accomplished by distillation using methyl benzoate or the methyl ester of p-toluic acid as an azeotropic agent.

Many manufacturers and consumers prefer to use more ecologically acceptable PBT. One method of making PBT more ecologically acceptable is to use recycled or scrap material in its manufacture. A particular challenge, however, in using recycle or scrap material is attaining properties comparable to the PBT derived from virgin material. There accordingly remains a need in the art for PBT derived at least in part from recycle or scrap material wherein the critical properties of the PBT are comparable to PBT derived from virgin material, and for compositions containing such PBT.

### BRIEF SUMMARY OF THE INVENTION

A flame retardant poly(butylene terephthalate) composition comprising:
(a) a poly(butylene terephthalate) copolymer reaction product of 1,4 butanediol and a dimethyl terephthalate residual composition comprising a dimethyl terephthalate derived from a terephthalic-containing polyester homopolymer, terephthalic-containing polyester copolymer, or a combination thereof; and from more than 0 to less than 3.6 wt.% of a residual component selected from dimethyl isophthalate, cyclohexane dimethanol, diethylene glycol, triethylene glycol, or a combination thereof; and
(b) a flame retarding amount of a brominated polycarbonate,
wherein the flame retardant composition has a heat deflection temperature ranging from 61°C to 74°C when measured at 1.82 MPa in accordance with ASTM Method D648.

According to one embodiment, in the flame retardant poly(butylene terephthalate) composition the dimethyl terephthalate residual composition comprises from more than 0 to less than or equal to 0.9 weight %of the residual component; and the flame retardant composition has a heat deflection temperature ranging from 150°C to 156°C when measured at 1.82 MPa in accordance with ASTM Method D648.

Another embodiment refers to a flame retardant poly(butylene terephthalate) composition comprising from 30 to 90 wt.% of the poly(butylene terephthalate) copolymer reaction product of 1,4-butanediol and the dimethyl terephthalate residual composition, comprising from more than 0 to less than 3.6 wt.% of a residual component selected from dimethyl isophthalate, cyclohexane dimethanol, diethylene glycol, triethylene glycol, or a combination thereof;
from 10 to 80 wt.% of a flame retardant brominated polycarbonate;
from 10 to 80 wt.% of a polycarbonate;
from more than 0 to 20 wt.% of an additional flame retardant;
from more than 0 to 2 wt.% of the antioxidant;
from more than 0 to 2 wt.% of a mold release agent;
from more than 0 to 2 wt.% of an anti-drip agent;
and from more than 0 to 2 wt.% of a quencher.

A method for the manufacture of the flame retardant poly(butylene terephthalate) compositions comprising melt blending the components of the composition.

Further disclosed are articles comprising the flame retardant poly(butylene terephthalate) compositions.

Methods of manufacturing an article, comprising shaping by extrusion, calendering, molding, or injection molding the flame retardant poly(butylene terephthalate) composition.

The above described and other features are exemplified by the following drawings, detailed description, and examples.

### BRIEF DESCRIPTION OF THE DRAWINGS

Figure 1 shows melting point as a function of the percentage of total impurities in the PBT made in Examples 1-10.
Figure 2 shows crystallization temperature as a function of the percentage of total impurities in the PBT made in Examples 1-10.
Figure 3 shows heat deflection temperature with a loading stress of 0.455 MPa as a function of the percentage of total impurities present in the dimethyl terephthalate used to make the PBT used in the stabilized PBT made in Examples 11-20.
Figure 4 shows heat deflection temperature with a loading stress of 1.82 MPa as a function of the percentage of total impurities present in the dimethyl terephthalate used to make the PBT used in the stabilized PBT made in Examples 11-20.
Figure 5 shows mold shrinkage measured in the parallel direction as a function of the percentage of total impurities present in the dimethyl terephthalate used to make the PBT used in the stabilized PBT made in Examples 11-20.
Figure 6 shows mold shrinkage measured in the perpendicular direction as a function of the percentage of total impurities present in the dimethyl terephthalate used to make the PBT used in the stabilized PBT made in Examples 11-20.
Figure 7 shows tensile elongation at break as a function of the percentage of total impurities present in the dimethyl terephthalate used to make the PBT used in the stabilized PBT made in Examples 11-20.
Figure 8 shows heat deflection temperature with a loading stress of 0.455 MPa as a function of the percentage of total impurities present in the dimethyl terephthalate used to make the PBT used in the flame retardant composition with a very high PBT content made in Examples 21-30.
Figure 9 shows heat deflection temperature with a loading stress of 1.82 MPa as a function of the percentage of total impurities present in the dimethyl terephthalate used to make the PBT used in the flame retardant composition with a very high PBT content made in Examples 21-30.
Figure 10 shows total energy measured by the biaxial impact test as a function of the percentage of total impurities present in the dimethyl terephthalate used to make the PBT used in the flame retardant composition with a very high PBT content made in Examples 21-30.
Figure 11 shows heat deflection temperature with a loading stress of 0.455 MPa as a function of the percentage of total impurities present in the dimethyl terephthalate composition used to make the PBT used in the impact modified composition with a polycarbonate / PBT blend using a high PBT content made in Examples 31-40.
Figure 12 shows heat deflection temperature with a loading stress of 1.82 MPa as a function of the percentage of total impurities present in the dimethyl terephthalate used to make the PBT used in the impact modified composition with a polycarbonate / PBT blend using a high PBT content made in Examples 31-40.
Figure 13 shows modulus of elasticity as a function of the percentage of total impurities present in the dimethyl terephthalate used to make the PBT used in the impact modified composition with a polycarbonate / PBT blend using a high PBT content made in Examples 31-40.

### DETAILED DESCRIPTION OF THE INVENTION

Our invention is based on the discovery that it is now possible to make a flame retardant poly(butylene terephthalate) (PBT) composition, specifically a flame retardant polycarbonate-PBT composition, where the PBT is derived at least in part from recycle or scrap polyesters. The PBT used in the flame retardant compositions has a highly useful and unexpected combination of desirable properties, including heat deflection temperature. In particular, the PBT is manufactured using a dialkyl terephthalate residual composition that is derived from a terephthalic acid-based polyester. It has been discovered that careful selection and regulation of certain amounts and/or types of impurities in the residual composition allows the manufacture of PBT from scrap or recycle polyesters that provides compositions having properties comparable to those using PBT derived from virgin monomers.

As used herein, the terms "recycle" or "scrap" are not intended to limit the source of the terephthalic acid-based polyester, as any terephthalic acid-based polyester can be used regardless of its source.

As used herein the singular forms "a," "an," and "the" include plural referents. The term "combination" is inclusive of blends, mixtures, alloys, reaction products. Unless defined otherwise, technical and scientific terms used herein have the same meaning as is commonly understood by one of skill. Compounds are described using standard nomenclature. The term "and a combination thereof' is inclusive of the named component and/or other components not specifically named that have essentially the same function.

Other than in the operating examples or where otherwise indicated, all numbers or expressions referring to quantities of ingredients, reaction conditions, used in the specification and claims are to be understood as modified in all instances by the term "about." Various numerical ranges are disclosed in this patent application. Because these ranges are continuous, they include every value between the minimum and maximum values. The endpoints of all ranges reciting the same characteristic or component are independently combinable and inclusive of the recited endpoint. Unless expressly indicated otherwise, the various numerical ranges specified in this application are approximations. The term "from more than 0 to" an amount means that the named component is present in some amount that is greater than 0, and up to and including the higher named amount.

The PBT composition comprises a PBT that has been manufactured using a terephthalic acid diester monomer derived from a terephthalic acid-containing homopolymer, a terephthalic-containing copolymer, or a combination thereof. For example, the PBT can be using a terephthalic acid diester monomer derived from a terephthalic acid-containing polyester, wherein the polyester can be a homopolymer, a copolymer, or a combination thereof. For convenience such polyester homopolymers, copolymers, and combination can be collectively referred to herein as a poly(hydrocarbylene terephthalate). Poly(hydrocarbylene terephthalate)s may contain units derived from dicarboxylic acids in addition to the terephthalic acid, for example isophthalic acid, adipic acid, glutaric acid, azelaic acid, sebacic acid, fumaric acid, and the various isomers of cyclohexane dicarboxylic acid. The poly(hydrocarbylene terephthalate)s can be a poly(alkylene terephthalate), wherein the alkylene group comprises 2 to 18 carbon atoms. Examples of alkylene groups include ethylene, 1,2-butylene, 1,3-butylene, 1,4-butylene, trimethylene, pentylene, hexylene, cyclohexylene, 1,4-cyclohexylene, and 1,4-cyclohexanedimethylene. A combination comprising at least one of the foregoing alkylene groups can be present. Poly(arylene terephthalate)s can also be used, for example poly(naphthalene terephthalate)s based on naphthalene dicarboxylic acids, including the 2,6-, 1,4-, 1,5-, or 2,7-isomers but the 1,2-, 1,3-, 1,6-, 1,7-, 1,8-, 2,3-, 2,4-, 2,5-, and/or 2,8-isomers. The poly(hydrocarbylene terephthalate)s can also have a combination of alkylene and arylene groups. In a specific embodiment, the poly(hydrocarbylene terephthalate) is poly(ethylene terephthalate). As described above, other alkylene or arylene groups can be present, in addition to residues derived from other dicarboxylic acids.

The residual composition comprising the diester monomer derived from the poly(hydrocarbylene terephthalate) can comprise a (C₁₋₃)alkyl ester of terephthalic acid, and in particular a dimethyl terephthalate (DMT). The dimethyl terephthalate residual composition can be made by any suitable process, provided that the resulting dimethyl terephthalate residual composition includes more than 0 to less than 5.1 wt.% of a residual component selected from dimethyl isophthalate, cyclohexane dimethanol, diethylene glycol, triethylene glycol, or a combinations thereof. In an embodiment, our compositions can be made by a process that involves depolymerization of a polyester by, for example, treatment of bulk poly(ethylene terephthalate) with super-heated methanol vapor in the presence of a suitable transesterification catalyst at atmospheric pressure, provided that the resulting dimethyl terephthalate residual composition includes more than 0 to less than 5.1 wt.% of a residual component selected from dimethyl isophthalate, cyclohexane dimethanol, diethylene glycol, triethylene glycol, or a combinations thereof. In another embodiment, poly(alkylene terephthalate, particularly poly(ethylene terephthalate), can alternatively be treated with steam at a temperature from about 200°C to about 450°C, where the steam-treated poly(ethylene terephthalate) is then reduced from a brittle solid product to a powder having a mean particles size of from about 0.0005 to 0.002 millimeters, after which the fine powder is atomized with a gaseous substance including inert gas and methanol vapor to form an aerosol. The aerosol is conducted through a reaction zone at a temperature of 250°C to 300°C in the presence of excess methanol vapors to produce DMT, provided that the resulting dimethyl terephthalate residual composition includes more than 0 to less than 5.1 wt.% of a residual component selected from dimethyl isophthalate, cyclohexane dimethanol, diethylene glycol, triethylene glycol, or a combinations thereof. Alternatively, poly(ethylene terephthalate) waste can be subdivided into dimensions between 4 and 35 mesh and treated at a temperature of 100°C to 300°C in the presence of methanol and acid catalysts to produce DMT, provided that the resulting dimethyl terephthalate residual composition includes more than 0 to less than 5.1 wt.% of a residual component selected from dimethyl isophthalate, cyclohexane dimethanol, diethylene glycol, triethylene glycol, or a combinations thereof. Scrap polyester can be dissolved in oligomers of ethylene glycol and terephthalate acid or dimethyl terephthalate, followed by passing super-heated methanol through the solution, where the ethylene glycol and dimethyl terephthalate are subsequently recovered overhead. In still another embodiment, our composition can be made by processes that use methanol vapor under pressurized conditions, provided that the resulting dimethyl terephthalate residual composition includes more than 0 to less than 5.1 wt.% of a residual component selected from dimethyl isophthalate, cyclohexane dimethanol, diethylene glycol, triethylene glycol, or a combinations thereof. The temperature at which the process to make the residual composition is practiced can vary. In an embodiment, when a process that uses methanol under pressure is used in conjunction with a transesterification catalyst, the process is practiced at a temperature ranging from 120-200 °C, or higher than 200°C.

The monomers produced from these processes can be purified, for example by techniques such as distillation, crystallization, and filtration. However, the cost of the purification steps can render the recovered monomers more expensive than virgin raw materials. Surprisingly, it has been found by the inventors hereof that only very specific levels of impurities can be present in the (C₁₋₃)alkyl terephthalic esters, in particular DMT, used to produce PBT, yielding PBT that has properties comparable to PBT produced from virgin monomers. Without being bound by theory, it is believed that at least a portion of the impurities are incorporated into the PBT backbone, resulting in a copolymer with altered properties.

Thus, a di(C₁₋₃)alkyl terephthalic ester residual composition, and a DMT residual composition in particular derived from a terephthalic-containing polyester homopolymer, terephthalic-containing copolymer, or a combination thereof and containing from more than 0 to less than 3.6 wt.% of a residual component selected from dimethyl isophthalate, cyclohexane dimethanol, diethylene glycol, triethylene glycol, and combinations thereof can be successfully used in the manufacture of PBT that can be used in an flame retardant polycarbonate-PBT composition having commercially valuable properties.

Methods for the manufacture of PBT from a di(C₁₋₃)alkyl terephthalic esters are known, and can be used to manufacture PBT from a di(C₁₋₃)alkyl terephthalic ester residual compositions, and in particular the DMT residual compositions, and 1,4-butanediol. For example, polyesters can be obtained by interfacial polymerization, melt-process condensation, or solution phase condensation in the presence of an acid catalyst. The catalyst facilitates the transesterification reaction, and can be selected from antimony compounds, tin compounds, titanium compounds, combinations thereof as well as many other metal catalysts and combinations of metal catalysts that have been disclosed in the literature. The amount of catalyst required to obtain an acceptable polymerization rate at the desired polymerization temperature will vary, and can be determined by experimentation. The catalyst amount can be 1 to 5000 ppm, or more. It is possible to prepare a branched polyester in which a branching agent, for example, a glycol having three or more hydroxyl groups or a trifunctional or multifunctional carboxylic acid has been incorporated. Furthermore, it is sometime desirable to have various concentrations of acid and hydroxyl end groups on the polyester, depending on the ultimate end use of the composition.

In general, the PBT manufactured from 1,4-butanediol and the DMT residue from more than 0 to less than 5.1, preferably less than 3.6 wt.% of a residual component selected from dimethyl isophthalate, cyclohexane dimethanol, diethylene glycol, triethylene glycol, and combinations thereof can have an intrinsic viscosity of 0.4 to 2.0 deciliters per gram (dL/g), specifically 0.5 to 1.5 dL/g, more specifically 0.6 to 1.2 dL/g, as measured in a 60:40 by weight phenol/1,1,2,2-tetrachloroethane mixture at 23°C. The PBT can have a weight average molecular weight of 10,000 to 200,000 Daltons, specifically 50,000 to 150,000 Daltons as measured by gel permeation chromatography (GPC). The polyester component can also comprise a mixture of different batches of PBT prepared under different process conditions in order to achieve different intrinsic viscosities and/or weight average molecular weights.

In a specific embodiment, the PBT reaction product of 1,4-butanediol and the DMT residue having from more than 0 to less than 5.1 wt.%, specifically less than 3.6 wt.%, less than 3.1 wt.%, less than 1.4 wt.% or less than 0.9 wt.%, of a residual component selected from dimethyl isophthalate, cyclohexane dimethanol, diethylene glycol, triethylene glycol, and combinations thereof can have a melting temperature that is within 1.5°C of the melting temperature of the same PBT that is the reaction product of virgin dimethyl terephthalate and 1,4-butanediol.

The PBT reaction product of 1,4-butanediol and the DMT residue having from more than 0 to less than 5.1 wt.%, specifically less than 3.6 wt.%, less than 3.1 wt.%, less than 1.4 wt.%, or less than 0.9 wt.%, of a residual component selected from dimethyl isophthalate, cyclohexane dimethanol, diethylene glycol, triethylene glycol, and combinations thereof butanediol can have a crystallization temperature that is within 7.4°C of the crystallization temperature of the same PBT that is the reaction product of virgin dimethyl terephthalate.

The PBT reaction product of the DMT residue and 1,4-butanediol can be used a component in flame retardant PBT compositions, in particular flame retardant polycarbonate-PBT compositions having a high percentage of PBT.

The flame retardant is a brominated polycarbonate. In general, the amount of bromine present in the brominated polycarbonate can be 1 to 45 wt.%, specifically 2 to 40 wt.%, more specifically 3 to 35 wt.%, based on the total weight of the brominated polycarbonate.

In an embodiment, the brominated polycarbonate is a copolycarbonate prepared from brominated and unbrominated dihydroxy compounds, which is described below. In this embodiment, the ratio of brominated units to unbrominated units in the copolycarbonate is selected to provide the targeted amount of bromine in the copolycarbonate. A specific brominated polycarbonate is a copolycarbonate comprising structural units derived from bisphenol A and tetrabromobisphenol A, which as produced by SABIC INNOVATIVE PLASTICS under the trade name LEXAN PC105B.

Brominated polycarbonates are often manufactured or provided as a combination with a non-brominated polycarbonate, which as used herein includes combinations with polycarbonate, copolycarbonate, and/or polyestercarbonate. Binary blends, ternary blends, and blends having more than three components may also be used. When a binary blend or ternary blend is used, one of the components may be present at 1 to 99 wt.% based on the total weight of the blend, specifically 20 to 90 wt.%, more specifically 30 to 80 wt.%, and yet more specifically 40 to 60 wt.% based on the total weight of the blend. When ternary blends or blends having more than three polymeric resins are used, the various components may be present in any desirable weight ratio to provide a ductile material.

Brominated polycarbonate is also inclusive of combinations comprising brominated carbonate oligomers. Such brominated carbonate oligomers comprise 1 to 80 wt.% bromine based on the total weight of the oligomer, specifically 10 to 70 wt.%, and more specifically 30 to 60 wt.%, and yet more specifically 40 to 55 wt.% bromine based on the total weight of the brominated oligomer. Exemplary brominated oligomers include those commercially available from Great Lakes Chemical, including for example those under the trade name GREAT LAKES BC-52, GREAT LAKES BC-52 HP, GREAT LAKES BC-58, and GREAT LAKES PE-68. When used in a blend, the brominated carbonate oligomer may be present in an amount of 1 to 55 wt.% based on the total weight of the blend, specifically 10 to 45 wt.%, and more specifically 20 to 35 wt.%. Thus, in an embodiment, the brominated polycarbonate comprises a blend of brominated carbonate oligomers and a polycarbonate free of bromine. The ratio of brominated carbonate oligomer to the polycarbonate free of bromine may be selected to provide an amount of bromine of 1 to 45 wt.%, specifically 10 to 40 wt.%, more specifically 15 to 35 wt.%, based on the total weight of the blend.

In another embodiment, the brominated polycarbonate comprises a blend of brominated carbonate oligomers and a copolycarbonate prepared from a brominated dihydroxy compound and a dihydroxy compound free of bromine. Within this embodiment, the ratio of brominated carbonate oligomer to the copolycarbonate may be chosen to provide an amount of bromine of 1 to 45 wt.%, specifically 10 to 40 wt.%, more specifically 15 to 35 wt.%, and yet more specifically 20 to 30 wt.% based on the total weight of the blend.

Optionally, a non-brominated polycarbonate can be added separately to the flame retardant PBT compositions. Suitable non-brominated polycarbonates for use in the flame retardant compositions are known in the art, for example compositions having repeating structural carbonate units of the formula (1): 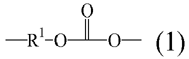 in which at least 60 percent of the total number of R¹ groups contain aromatic organic groups and the balance thereof are aliphatic, alicyclic, or aromatic groups. In an embodiment, each R¹ is a C₆₋₃₀ aromatic group, that is, contains at least one aromatic moiety. R¹ can be derived from a dihydroxy compound of the formula HO-R¹-OH, in particular a dihydroxy compound of formula (2):

HO-A¹-Y¹-A²-OH (2)

wherein each of A¹ and A² is a monocyclic divalent aromatic group and Y¹ is a single bond or a bridging group having one or more atoms that separate A¹ from A². In an exemplary embodiment, one atom separates A¹ from A². Specifically, each R¹ can be derived from a dihydroxy aromatic compound of formula (3) 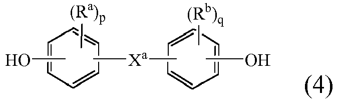 wherein R^{a} and R^{b} each represent a halogen or C₁₋₁₂ alkyl group and can be the same or different; and p and q are each independently integers of 0 to 4. Also in formula (6), X^{a} represents a bridging group connecting the two hydroxy-substituted aromatic groups, where the bridging group and the hydroxy substituent of each C₆ arylene group are disposed ortho, meta, or para (specifically para) to each other on the C₆ arylene group. In an embodiment, the bridging group X^{a} is single bond, -O-, -S-, -S(O)-, -S(O)₂-, -C(O)-, or a C₁₋₁₈ organic group. The C₁₋₁₈ organic bridging group can be cyclic or acyclic, aromatic or non-aromatic, and can further comprise heteroatoms such as halogens, oxygen, nitrogen, sulfur, silicon, or phosphorous. The C₁₋₁₈ organic bridging group can be disposed such that the C₆ arylene groups connected thereto are each connected to a common alkylidene carbon or to different carbons of the C₁₋₁₈ organic bridging group. In an embodiment, p and q is each 1, and R^{a} and R^{b} are each a C₁₋₃ alkyl group, specifically methyl, disposed meta to the hydroxy group on each arylene group.

In an embodiment, X^{a} is a substituted or unsubstituted C₃₋₁₈ cycloalkylidene, a C₁₋₂₅ alkylidene of formula -C(R^{c})(R^{d})- wherein R^{c} and R^{d} are each independently hydrogen, C₁₋₁₂ alkyl, C₁₋₁₂ cycloalkyl, C₇₋₁₂ arylalkyl, C₁₋₁₂ heteroalkyl, or cyclic C₇₋₁₂ heteroarylalkyl, or a group of the formula -C(=R^{e})- wherein R^{e} is a divalent C₁₋₁₂ hydrocarbon group. Exemplary groups of this type include methylene, cyclohexylmethylene, ethylidene, neopentylidene, and isopropylidene, as well as 2-[2.2.1]-bicycloheptylidene, cyclohexylidene, cyclopentylidene, cyclododecylidene, and adamantylidene.

Other useful aromatic dihydroxy compounds of the formula HO-R'-OH include compounds of formula (4) 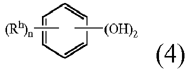 herein each R^{h} is independently a halogen atom, a C₁₋₁₀ hydrocarbyl such as a C₁₋₁₀ alkyl group, a halogen-substituted C₁₋₁₀ alkyl group, a C₆₋₁₀ aryl group, or a halogen-substituted C_{6- 10} aryl group, and n is 0 to 4. The halogen is usually bromine.

Some illustrative examples of specific aromatic dihydroxy compounds include the following: 4,4'-dihydroxybiphenyl, 1,6-dihydroxynaphthalene, 2,6-dihydroxynaphthalene, bis(4-hydroxyphenyl)methane, bis(4-hydroxyphenyl)diphenylmethane, bis(4-hydroxyphenyl)-1 -naphthylmethane, 1,2-bis(4-hydroxyphenyl)ethane, 1,1 -bis(4-hydroxyphenyl)-1 -phenylethane, 2-(4-hydroxyphenyl)-2-(3-hydroxyphenyl)propane, bis(4-hydroxyphenyl)phenylmethane, 2,2-bis(4-hydroxy-3-bromophenyl)propane, 1,1-bis (hydroxyphenyl)cyclopentane, 1,1-bis(4-hydroxyphenyl)cyclohexane, 1,1 -bis(4-hydroxyphenyl)isobutene, 1,1 -bis(4-hydroxyphenyl)cyclododecane, trans-2,3-bis(4-hydroxyphenyl)-2-butene, 2,2-bis(4-hydroxyphenyl)adamantine, alpha, alpha'-bis(4-hydroxyphenyl)toluene, bis(4-hydroxyphenyl)acetonitrile, 2,2-bis(3-methyl-4-hydroxyphenyl)propane, 2,2-bis(3-ethyl-4-hydroxyphenyl)propane, 2,2-bis(3-n-propyl-4-hydroxyphenyl)propane, 2,2-bis(3-isopropyl-4-hydroxyphenyl)propane, 2,2-bis(3-sec-butyl-4-hydroxyphenyl)propane, 2,2-bis(3-t-butyl-4-hydroxyphenyl)propane, 2,2-bis(3-cyclohexyl-4-hydroxyphenyl)propane, 2,2-bis(3-allyl-4-hydroxyphenyl)propane, 2,2-bis(3-methoxy-4-hydroxyphenyl)propane, 2,2-bis(4-hydroxyphenyl)hexafluoropropane, 1,1-dichloro-2,2-bis(4-hydroxyphenyl)ethylene, 1,1-dibromo-2,2-bis(4-hydroxyphenyl)ethylene, 1,1-dichloro-2,2-bis(5-phenoxy-4-hydroxyphenyl)ethylene, 4,4'-dihydroxybenzophenone, 3,3-bis(4-hydroxyphenyl)-2-butanone, 1,6-bis(4-hydroxyphenyl)-1,6-hexanedione, ethylene glycol bis(4-hydroxyphenyl)ether, bis(4-hydroxyphenyl)ether, bis(4-hydroxyphenyl)sulfide, bis(4-hydroxyphenyl)sulfoxide, bis(4-hydroxyphenyl)sulfone, 9,9-bis(4-hydroxyphenyl)fluorine, 2,7-dihydroxypyrene, 6,6'-dihydroxy-3,3,3',3'- tetramethylspiro(bis)indane ("spirobiindane bisphenol"), 3,3-bis(4-hydroxyphenyl)phthalide, 2,6-dihydroxydibenzo-p-dioxin, 2,6-dihydroxythianthrene, 2,7-dihydroxyphenoxathin, 2,7-dihydroxy-9,10-dimethylphenazine, 3,6-dihydroxydibenzofuran, 3,6-dihydroxydibenzothiophene, and 2,7-dihydroxycarbazole, resorcinol, substituted resorcinol compounds such as 5-methyl resorcinol, 5-ethyl resorcinol, 5-propyl resorcinol, 5-butyl resorcinol, 5-t-butyl resorcinol, 5-phenyl resorcinol, 5-cumyl resorcinol, 2,4,5,6-tetrafluoro resorcinol, 2,4,5,6-tetrabromo resorcinol, or the like; catechol; hydroquinone; substituted hydroquinones such as 2-methyl hydroquinone, 2-ethyl hydroquinone, 2-propyl hydroquinone, 2-butyl hydroquinone, 2-t-butyl hydroquinone, 2-phenyl hydroquinone, 2-cumyl hydroquinone, 2,3,5,6-tetramethyl hydroquinone, 2,3,5,6-tetra-t-butyl hydroquinone, 2,3,5,6-tetrafluoro hydroquinone, 2,3,5,6-tetrabromo hydroquinone, as well as combinations comprising at least one of the foregoing dihydroxy compounds.

Specific examples of bisphenol compounds include 1,1-bis(4-hydroxyphenyl) methane, 1,1-bis(4-hydroxyphenyl) ethane, 2,2-bis(4-hydroxyphenyl) propane (hereinafter "bisphenol A" or "BPA"), 2,2-bis(4-hydroxyphenyl) butane, 2,2-bis(4-hydroxyphenyl) octane, 1,1-bis(4-hydroxyphenyl) propane, 1,1-bis(4-hydroxyphenyl) n-butane, 2,2-bis(4-hydroxy-1-methylphenyl) propane, 1,1-bis(4-hydroxy-t-butylphenyl) propane, 3,3-bis(4-hydroxyphenyl) phthalimidine, 2-phenyl-3,3-bis(4-hydroxyphenyl) phthalimidine (PPPBP), and 1,1-bis(4-hydroxy-3-methylphenyl)cyclohexane (DMBPC). Combinations comprising at least one of the foregoing dihydroxy compounds can also be used. In one specific embodiment, the polycarbonate is a linear homopolymer derived from bisphenol A, in which each of A¹ and A² is p-phenylene and Y¹ is isopropylidene in formula (4).

In a specific embodiment, the polycarbonate can be a linear homopolymer derived from bisphenol A, in which each of A¹ and A² is p-phenylene and Y¹ is isopropylidene. The polycarbonates generally can have an intrinsic viscosity, as determined in chloroform at 25°C, of 0.3 to 1.5 deciliters per gram (dl/g), specifically 0.45 to 1.0 dl/g. The polycarbonates can have a weight average molecular weight (Mw) of 10,000 to 100,000 g/mol, as measured by gel permeation chromatography (GPC) using a crosslinked styrene-divinyl benzene column, at a sample concentration of 1 milligram per milliliter, and as calibrated with polycarbonate standards.

In an embodiment, the polycarbonate can have a melt volume flow rate (often abbreviated MVR) measures the rate of extrusion of a thermoplastics through an orifice at a prescribed temperature and load. Polycarbonates useful for the formation of articles can have an MVR, measured at 300°C under a load of 1.2 kg according to ASTM D1238-04 or ISO 1133, of 0.5 to 80 cubic centimeters per 10 minutes (cc/10 min). In a specific embodiment, where a polycarbonate is used in addition to the polyestercarbonate, the polycarbonate (or a combination of polycarbonates, i.e., a polycarbonate composition) can have an MVR measured at 300°C under a load of 1.2 kg according to ASTM D1238-04 or ISO 1133, of 5 to 35 cc/10 min, specifically 10 to 35 cc/10 min, and more specifically 25 to 35 cc/10 min.

The flame retardant compositions comprise from 30 to 90 wt.% of the polybutylene terephthalate copolymer reaction product of the dimethyl terephthalate residual composition of as described above and butane diol; from 10 to 80 wt.% of the polycarbonate; and from more than 0 to 25 wt.% of the impact modifier, based on the total weight of the flame retardant composition. In a specific embodiment, the flame retardant compositions comprise from 30 to 90 wt.% of the polybutylene terephthalate copolymer reaction product of the dimethyl terephthalate residual composition of our invention and butane diol; from 10 to 69 wt.% of the polycarbonate; and from 1 to 22 wt.% of the impact modifier, based on the total weight of the flame retardant composition. In another specific embodiment, the flame retardant compositions comprise from 40 to 80 wt.% of the polybutylene terephthalate copolymer reaction product of the dimethyl terephthalate residual composition of our invention and butane diol; from 10 to 59 wt.% of the polycarbonate; and from 1 to 15 wt.% of the impact modifier, based on the total weight of the flame retardant composition.

With the proviso that desired properties, such as mold shrinkage, tensile elongation, heat deflection temperature, are not adversely affected, the thermoplastic compositions comprising PBT manufactured from the di(C₁₋₃)alkyl terephthalic ester residue, specifically the DMT residue, can optionally further comprise other known additives used in thermoplastic polyester compositions such as non-reinforcing fillers, stabilizers such as antioxidants, thermal stabilizers, radiation stabilizers, and ultraviolet light absorbing additives, as well as mold release agents, plasticizers, quenchers, lubricants, antistatic agents, dyes, pigments, laser marking additives, and processing aids. A combination comprising one or more of the foregoing or other additives can be used. When used, the additives are each generally present in an amount of 0.01 to 5 wt.%, specifically 0.05 to 2 wt.%.. A specific example of an additive combination is an antioxidant such as a hindered phenol stabilizer, a quencher, an anti-drip agent, and a waxy mold release agent such as pentaerythritol tetrastearate, each present in an amount from more than 0, specifically 0.1 to 2 wt.%, based on the total weight of the flame retardant composition.

Anti-dripping agents prevent or retard the resin from dripping while the resin is subjected to burning conditions. Examples of such agents include silicone oils, silica (which also serves as a reinforcing filler), asbestos, and fibrillating-type fluoropolymers. Examples of fluoropolymers include fluorinated polyolefins such as poly(tetrafluoroethylene), tetrafluoroethylene/hexafluoropropylene copolymers, tetrafluoroethylene/ethylene copolymers, poly(vinylidene fluoride), poly(chlorotrifluoroethylene), and mixtures comprising at least one of the foregoing anti-dripping agents. A preferred anti-dripping agent is poly(tetrafluoroethylene) encapsulated by a poly(styrene-co-acrylonitrile) (SAN) copolymer. The anti-dripping agent is present in an amount of 0.01 to 5 wt.%, specifically from 0.05 to 2 wt.%, and more specifically 0.1 to 1 wt.%, based on the total weight of the flame retardant composition.

One or more additional flame retardants can also be present in the impact modified compositions, selected from sulfonated salts, brominated compounds, antimony flame retardants, phosphorus-containing flame retardants, nitrogen-containing flame retardants, phosphinate salts, and combinations thereof.

Inorganic flame retardants can be used, for example salts of C₂₋₁₆ alkyl sulfonate salts such as potassium perfluorobutane sulfonate (Rimar salt), potassium perfluorooctane sulfonate, tetraethyl ammonium perfluorohexane sulfonate, and potassium diphenylsulfone sulfonate; salts formed by reacting for example an alkali metal or alkaline earth metal (for example lithium, sodium, potassium, magnesium, calcium and barium salts) and an inorganic acid complex salt, for example, an oxo-anion, such as alkali metal and alkaline-earth metal salts of carbonic acid, such as Na₂CO₃, K₂CO₃, MgCO₃, CaCO₃, and BaCO₃ or fluoro-anion complexes such as Li₃AlF₆, BaSiF₆, KBF₄, K₃AlF₆, KAlF₄, K₂SiF₆, and/or Na₃AlF₆ or the like. When present, inorganic flame retardant salts can be present in amounts of 0.1 to 5 wt.%, based on the total weight of the composition.

Brominated organic compounds contain bromine atoms directly bonded to an organic moiety, for example an aromatic or cycloaliphatic ring, for example compounds derived from brominated Bisphenol A, tetrabromophthalic anhydride, and pentabromocyclohexane, pentabromochlorocyclohexane, hexabromocyclohexane, 1,2-dibromo-4-(1,2-dibromoethyl)-cyclohexane, tetrabromocyclooctane, hexabromocyclooctane, hexabromocyclododecane, chloroparaffins, and analogous bromine- or chlorine- containing aliphatic or cycloaliphatic compounds. Brominated polycarbonates include copolycarbonates prepared from brominated and unbrominated dihydroxy compounds. The ratio of brominated units to unbrominated units in the copolycarbonate is chosen to provide an amount of bromine of 1 to 45 wt.%, based on the total weight of the copolycarbonate. An exemplary brominated polycarbonate is a copolycarbonate comprising structural units derived from bisphenol A and tetrabromobisphenol A. Brominated carbonate oligomers can be used, which comprise 1 to 80 wt.% bromine based on the total weight of the oligomer, including for example those available from Great Lakes Chemical under the trade name GREAT LAKES BC-52, GREAT LAKES BC-52 HP, GREAT LAKES BC-58, and GREAT LAKES PE-68.

Organic flame retardants that can be used include various phosphorus-containing compounds, in particular aromatic phosphorus-containing compounds and brominated or chlorinated organic compounds. Organic compounds containing phosphorus-nitrogen bonds can be used, as well compounds such as phosphonate, phosphinate, phosphite, phosphine oxide, and phosphate esters can be used, for example triethyl phosphate, triphenyl phosphate, tris(dichloropropyl) phosphate; tris(2-chloroethyl)phosphate; tris(dibromopropyl) phosphate; tris(bromochloropropyl)phosphate.

The flame retardant composition can include a flame retarding quantity of one or a mixture of nitrogen-containing flame retardants such as triazines, guanidines, cyanurates, and isocyanurates. Exemplary triazines have the formula: 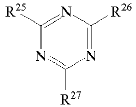 wherein R²⁵, R²⁶, and R²⁷ are independently C₁-C₁₂ alkyl, C₁-C₁₂ alkoxy, C₆-C₁₂ aryl, amino, C₁-C₁₂ alkyl-substituted amino, or hydrogen. Highly preferred triazines include 2,4,6-triamine-1,3, 5-triazine (melamine, CAS Reg. No. 108-78-1), melamine derivatives, melam, melem, melon, ammeline (CAS Reg. No. 645-92-1), ammelide (CAS Reg. No. 645-93-2), 2-ureidomelamine, acetoguanamine (CAS Reg. No. 542-02-9), benzoguanamine (CAS Reg. No. 91-76-9). Salts/adducts of these compounds with boric acid or phosphoric acid can be used in the composition. Examples include melamine pyrophosphate and melamine polyphosphate (CAS Reg. No. 218768-84-4). Specific cyanurate/isocyanurate compounds include salts/adducts of the triazine compounds with cyanuric acid, such as melamine cyanurate and any mixtures of melamine salts. Specific guanidine compounds include guanidine; aminoguanidine; and their salts and adducts with boric acid, carbonic acid, phosphoric acid, nitric acid, sulfuric acid; and mixtures comprising at least one of the foregoing guanidine compounds. The nitrogen-containing flame retardant can be present in the flame retardant composition in an amount from 1 to 25 wt.%, based on the total weight of the composition. From 1 to 25 wt.% of a melamine polyphosphate, melamine cyanurate, melamine pyrophosphate, and/or melamine phosphate, based on the total weight of the composition can be used.

A flame retarding quantity of one or more phosphinic acid or diphosphinic acid salts can be used. The phosphinates and diphosphinates include those set forth in U.S. Patent No. 6,255,371 to Schosser et al. The phosphinates are of the formula [(R¹)(R²)(PO)-O]m⁻ M^{m+} (I), the formula (II) [(O-POR¹)(R³)(POR²-O)]²⁻n M^{m+} × (II), and/or polymers comprising formula (I) or (II), wherein R¹ and R² are identical or different and are H, C₁-C₆ linear or branched alkyl, or C₆-C₁₀ aryl; and R³ is C₁-C₁₀, linear or branched alkylene, C₆-C₁₀ arylene, C₇-C₁₁ alkylarylene, or C₇-C₁₁ arylalkylene; M is an alkaline earth metal, alkali metal, Al, Ti, Zn, Fe, or B; m is 1, 2, 3 or 4; n is 1, 2, or 3; and x is 1 or 2.

In an embodiment R¹ and R² are the same and are C₁-C₆-linear or branched alkyl, or phenyl; R³ is C₁-C₁₀-linear or branched alkylene, C₆-C₁₀-arylene, -alkylarylene or-arylalkylene; M is magnesium, calcium, aluminum, zinc, or a combination thereof; m is 1, 2 or 3; n is 1, 2 or 3; and x is 1 or 2. In a specific embodiment M is aluminum. Specifically R¹ and R² can be H. When R¹ and R² are H, the phosphinate of formula (I) is a hypophosphite. Examples of hypophosphites are calcium hypophosphite, aluminum hypophosphite. Specific examples of phosphinate (I) are aluminum diethylphosphinate (DEPAL) and zinc diethylphosphinate (DEPZN). The amount of phosphinate, diphosphinate, or polymers thereof, in the thermoplastic polyester composition can be from 1 to 35 wt.%, specifically from 5 to 20 wt.%, and more specifically from 10 to 15 wt.%, based on the total weight of the flame retardant composition.

Adjuncts of flame retardants can also be used, for example an optional charring polymer. A charring polymer is a polymer that has not more than 85% weight loss at 400°C to 500°C upon heating under nitrogen using a thermogravimetric analysis (TGA) at a heating rate of 20°C per minute. Typical charring polymers include polyetherimides, poly(phenylene ether), poly(phenylenesulfide), polysulfones, polyethersulfones, poly(phenylenesulfide oxide) (PPSO), and polyphenolics (e.g., novolacs). The charring polymer can be present in an amount from 0.1 to 15 wt.%, based on the total weight of the thermoplastic polyester composition. In a specific embodiment, a polyetherimide is present, specifically an aromatic polyetherimide. When present, the polyetherimide can be in an amount from more than 0 to 25 wt. %, specifically 0.1 to 25 wt. %, even more specifically from 2 to 8 wt. %, each based on the total weight of the flame retardant composition.

The thermoplastic PBT composition can optionally comprise a reinforcing fiber. Any rigid reinforcing fiber can be used, for example, a glass fiber, a carbon fiber, a metal fiber, a ceramic fiber or whisker. The fibers can be chopped or continuous.

In some applications it can be desirable to treat the surface of the fiber with a chemical coupling agent to improve adhesion to the polyester in the thermoplastic polyester composition. Examples of useful coupling agents are alkoxy silanes and alkoxy zirconates. Amino, epoxy, amide, or thio functional alkoxy silanes are especially useful. Fiber coatings with high thermal stability are preferred to prevent decomposition of the coating, which could result in foaming or gas generation during processing at the high melt temperatures required to form the compositions into articles.

The fibers can have diameters of 6 to 30 micrometers. The reinforcing fibers can be provided in the form of monofilaments or multifilaments and can be used either alone or in combination with other types of fiber, through, for example, co-weaving or core/sheath, side-by-side, orange-type or matrix and fibril constructions, or by other methods known to one skilled in the art of fiber manufacture. Suitable cowoven structures include, for example, glass fiber-carbon fiber, carbon fiber-aromatic polyimide (aramid) fiber, and aromatic polyimide fiberglass fiber or the like. Reinforcing fibers may be supplied in the form of, for example, rovings, woven fibrous reinforcements, such as 0-90 degree fabrics or the like; nonwoven fibrous reinforcements such as continuous strand mat, chopped strand mat, tissues, papers and felts or the like; or three-dimensional reinforcements such as braids.

In an embodiment, the reinforcing fiber is a glass fiber. An exemplary glass fiber is one that is relatively soda free, specifically fibrous glass filaments comprising lime-alumino-borosilicate glass, which is also known as "E" glass. In a specific embodiment the glass fiber has a diameter of 10 to 13 micrometers. The glass fibers can be flat. Exemplary flat glass fibers have a modulus of greater than or equal to 6,800 megaPascals (MPa), and can be chopped or continuous. The flat glass fiber can have various cross-sections, for example, trapezoidal, rectangular, or square. In preparing the molding compositions it is convenient to use a glass fiber in the form of chopped strands having an average length of from 0.1 mm to 10 mm, and having an average aspect ratio of 2 to 5. In articles molded from the compositions on the other hand shorter lengths will typically be encountered because during compounding considerable fragmentation can occur. When used, the glass fiber is present in an amount of 0.1 to 4.5 wt.%, more specifically 0.5 to 4 wt.%, and still more specifically 1 to 2.5 wt.%, based on the weight of the thermoplastic PBT composition. When amounts less than 0.1 wt.% are used, there is no improvement in flexural modulus or tensile strength (tensile stress at yield). When amounts greater than or equal to 5 wt.% are used, the thermoplastic polyester composition will not meet the UL94 V0 performance standard at a thickness of 0.8 mm and will not be sufficiently ductile.

The thermoplastic PBT compositions can optionally comprise a non-fibrous inorganic filler, which can impart additional beneficial properties to the compositions such as thermal stability, increased density, stiffness, and/or texture. Non-fibrous inorganic fillers include, alumina, amorphous silica, alumino silicates, mica, clay, talc, glass flake, glass microspheres, metal oxides such as titanium dioxide, zinc sulfide, ground quartz. The amount of non-fibrous filler can be in a range of between 0 wt.% and 50 wt.% based on the weight of the total composition.

The thermoplastic PBT compositions can optionally comprise both a reinforcing fiber and a platy filler. Combinations of glass fibers, carbon fibers or ceramic fibers with a platy filler, for example mica or flaked glass, can give enhanced properties. In general, the flat, plate-like filler has a length and width at least ten times greater than its thickness, where the thickness is from 1 to 1000 microns. Combinations of rigid fibrous fillers with flat, plate-like fillers can reduce warp of the molded article.

The thermoplastic compositions comprising PBT manufactured from the di(C₁₋₃)alkyl terephthalic ester residue, specifically the DMT residue, can be prepared by blending the components of the composition so that they are homogeneously dispersed in a continuous matrix of the polyester. A number of blending processes can be used. In an exemplary process, the PBT, other optional polymers, and/or other additives are mixed in an extrusion compounder and extruded to produce molding pellets. In another process, the components, including any reinforcing fibers and/or other additives, are mixed with the PBT by dry blending, fluxed on a mill and either comminuted or extruded and chopped. The components can also be mixed and directly molded, e.g. by injection or transfer molding. All of the components are dried as much as possible prior to blending. In addition, compounding is carried out with as short a residence time in the machine as possible and at the lowest possible temperature to minimize loss of components by decomposition or volatilization. The temperature is carefully controlled, and friction heat is utilized.

In an embodiment, the components are pre-compounded, pelletized, and then molded. Pre-compounding can be carried out in known equipment. For example, after predrying the polyester composition (e.g., for four hours at 120°C), a single screw extruder can be fed with a dry blend of the ingredients, the screw employed having a long transition section to ensure proper melting. Alternatively, a twin-screw extruder with intermeshing corotating screws can be fed with polyester and other components at the feed port and reinforcing fibers (and other additives) can optionally be fed downstream. In either case, a generally suitable melt temperature is 230°C to 300°C. The pre-compounded components can be extruded and cut up into molding compounds such as granules, pellets, by standard techniques. The granules or pellets can then be molded in any known equipment used for molding thermoplastic compositions, such as a Newbury or van Dorn type injection-molding machine with cylinder temperatures at 230°C to 280°C, and mold temperatures at 55°C to 95°C. The granules and pellets can also be extruded into films or sheets. The articles formed by molding or extrusion of the thermoplastic polyester compositions possess an excellent balance of flame retardance, ductility, tensile strength and flexural modulus.

The thermoplastic compositions comprising PBT manufactured from the di(C₁₋₃)alkyl terephthalic ester residue, specifically the DMT residue, can have excellent flame retardancy when molded into either thick or thin articles. A set of test conditions commonly accepted and used as a standard for flame retardancy is set forth in Underwriters Laboratories, Inc. Bulletin 94, which prescribes specific conditions for rating the self-extinguishing characteristics of a material. A 0.8 mm thick molded article comprising the thermoplastic polyester composition has a UL-94 flammability rating of V0 or V1.

An article molded from the thermoplastic compositions comprising PBT manufactured from the di(C₁₋₃)alkyl terephthalic ester residue, specifically the DMT residue having from more than 0 to less than 5.1 wt.%, less than 3.6 wt.%, less than 3.1 wt.%, less than 1.4 wt.%, or less than 0.9 wt.%, of a residual component selected from dimethyl isophthalate, cyclohexane dimethanol, diethylene glycol, triethylene glycol, and combinations thereof, can have a melt volume ratio (MVR) of 10 to 70 cm³/ 10 minute, more specifically 15 to 50 cm³/ 10 minute, measured in accordance with ISO11443 at 250°C and 2.16 kg.

Still further in an embodiment, an article molded from the thermoplastic compositions comprising PBT manufactured from the di(C₁₋₃)alkyl terephthalic ester residue, specifically the DMT residue having from more than 0 to less than 5.1 wt.% , specifically less than 3.6 wt.%, less than 3.1 wt.%, less than 1.4 wt.%, or less than 0.9 wt.%, of a residual component selected from dimethyl isophthalate, cyclohexane dimethanol, diethylene glycol, triethylene glycol, and combinations thereof, of the residual component, a brominated polycarbonate, and optionally one or more of an antioxidant, a mold release, an anti-drip agent, and a quencher has a heat deflection temperature from 61°C to 74°C when measured at 1.82 MPa in accordance with ASTM method D648. The foregoing composition can further optionally comprise a non-brominated polycarbonate, an additional flame retardant, and an anti-drip agent.

Still further in an embodiment, an article molded from the thermoplastic compositions comprising PBT manufactured from the di(C₁₋₃)alkyl terephthalic ester residue, specifically the DMT residue having from more than 0 to less than 1.4 wt.%, or less than 0.9 wt.%, of a residual component selected from dimethyl isophthalate, cyclohexane dimethanol, diethylene glycol, triethylene glycol, and combinations thereof, of the residual component, a polycarbonate, and optionally one or more of an antioxidant, a mold release, an anti-drip agent, and a quencher has a heat deflection temperature from 150°C to 156°C when measured at 0.455 MPa in accordance with ASTM method D648. The foregoing composition can further optionally comprise a non-brominated polycarbonate, an additional flame retardant, and an anti-drip agent.

Still further in an embodiment, an article molded from the thermoplastic compositions comprising PBT manufactured from the di(C₁₋₃)alkyl terephthalic ester residue, specifically the DMT residue having from more than 0 to less than 5.1 wt.% , specifically less than 3.6 wt.%, less than 3.1 wt.%, less than 1.4 wt.%, or less than 0.9 wt.%, of a residual component selected from dimethyl isophthalate, cyclohexane dimethanol, diethylene glycol, triethylene glycol, and combinations thereof, of the residual component, a brominated polycarbonate, and optionally one or more of an antioxidant, a mold release, an anti-drip agent, and a quencher has a heat deflection temperature from 61°C to 74°C when measured at 1.82 MPa in accordance with ASTM method D648. The foregoing composition can further optionally comprise a non-brominated polycarbonate, an additional flame retardant, and an anti-drip agent.

One or more of the foregoing properties can be achieved by a thermoplastic polyester composition that consists essentially of the PBT derived from a DMT residue the DMT residue having from more than 0 to less than 3.6 wt.%, less than 3.1 wt.%, less than 1.4 wt.%, or less than 0.9 wt.% of the residual component, a brominated polycarbonate, an antioxidant, a mold release, an anti-drip agent, a quencher and optionally a non-brominated polycarbonate, an additional flame retardant, and an anti-drip agent. Further, one or more of the foregoing properties can be achieved by a thermoplastic polyester composition that consists of the PBT derived from a DMT residue having from 0 to less than 3.6 wt.%, less than 3.1 wt.%, less than 1.4 wt.%, or less than 0.9 wt.% of the residual component, a brominated polycarbonate, antioxidant, mold release, anti-drip agent, quencher and optionally a non-brominated polycarbonate, additional flame retardant, and anti-drip agent.

Also disclosed are extruded, shaped, or molded articles comprising the PBT manufactured from the di(C₁₋₃)alkyl terephthalic ester residue, specifically the DMT residue, such as electric and electronic parts, including, for example, connectors, fans used in electronic devices such as computers, circuit breakers, lamp holders, fusers, power distribution box, enclosures, housings, and power plugs. A method of forming an article comprises extruding, calendering, molding, or injection molding the thermoplastic polyester composition to form the article. A specific article is a housing, in particular a housing for an electrical connector wherein at least one surface comprises the flame retardant composition.

Advantageously, our invention provides previously unavailable benefits, including thermoplastic PBT compositions containing PBT made from a DMT residual composition derived at least in part from a scrap or recycle polyester, yet desirable properties, including Tₘ, T_{c}, and heat deflection temperature that are the same as or similar to compositions containing PBT derived from virgin DMT. In addition, flame retardant compositions can be manufactured having desirable HDT properties.

The invention is further illustrated by the following non-limiting examples.

### EXAMPLES

The materials shown in Table 1 were used in the Examples.

| Table 1. | | |
|---|---|---|
| Component | Description | Source |
| 1,4-Butanediol | Monomer | BASF |
| Cyclohexane dimethanol | Co-monomer (impurity) | Eastman |
| Dimethyl isophthalate | Co-monomer (impurity) | Fisher scientific |
| Dimethyl terephthalate | Monomer | Invista |
| Tetra(isopropyl) titanate | Catalyst | DuPont |
| Triethylene glycol | Co-monomer (impurity) | Fisher Scientific |
| Diethylene glycol | Co-monomer (impurity) | Fisher Scientific |
| Poly(butylene terephthalate) | PBT from virgin DMT | Sabic Innovative Plastics |
| Hindered phenol stabilizer | Antioxidant | Chemtura |
| Hydrocarbon wax release | Mold release | Astor |
| Brominated polycarbonate | Flame retardant polymer | Sabic Innovative Plastics |
| Polycarbonate | Bisphenol A based Polymer | Sabic Innovative Plastics |
| Antimony oxide concentrate | Flame retardant | Industrial Plastics |
| Low density polyethylene | Mold release | Exxon Mobil |
| SAN encapsulated PTFE intermediate | Anti-drip agent | Sabic Innovative Plastics |
| Zinc phosphate | Quencher | Halox |
| Pentaerythritol beta-lauryl thiopropionate | Release agent | Chemtura |
| Anhydrous monosodium phosphate | Quencher | Innophos |
| MBS Impact Modifier | Methacrylate-butadiene-styrene | Dow Advanced Chemicals |
| 2-(2'hydroxy-5-T-octylphenyl)-benzotriazole | UV stabilizer | Americhem |

### Property Testing Procedures

The melting point, Tₘ, and the crystallization temperature, T_{c}, of the polymers were measured by DSC with a 20 °C/min heating rate. Tₘ was measured on the second heating cycle.

Molecular weight data was measured by gel permeation chromatography using polystyrene standards with a 2000 D cutoff.

Melt volume rate (MVR) of the polymers (dried for 2 hr at 120 °C prior to measurement) was measured according to ISO 1133 method with a dwelling time of 300 s a 0.0825 inch (2.1 mm) orifice at 250 °C with an applied load of 2.16 kg.

Melt viscosity (MV) of the polymer was measured by ISO 11443. A multipoint method is used to generate a melt viscosity curve as a function of shear rate.

Intrinsic viscosity (IV) data was obtained for the polymer.

Color data for the polymer was acquired by diffuse reflectance on a Gretag Macbeth Color-Eye 7000A with D65 illumination, 10° observer, CIE L*, a*, b*, specular included, UV included, large lens position, large aperture.

Flexural properties were measured using the ASTM D790 method using bar dimensions of 127 x 12.7 x 3.2 mm, a support span of 50.8 mm, and a test speed of 1.27 mm/min.

Heat Deflection Temperature was tested on five bars having the dimensions 127 x 12.7 x 3.2 mm using ASTM method D648 that uses a loading stress of 0.455 or 1.82 MPa with no prior annealing.

Notched Izod impact testing was done on 76.2 x 12.7 x 3.2 mm bars using ASTM method D256. Bars were notched prior to oven aging; samples were tested at room temperature (23 °C).

Un-notched Izod impact testing was done on 76.2 x 12.7 x 3.2 mm bars using ASTM method D4812. Samples were tested at room temperature (23 °C).

Biaxial impact testing, sometimes referred to as instrumented impact testing, was done as per ASTM D3763 using 101.6 x 3.2 mm molded discs with a test velocity of 3.3 m/s. The total energy absorbed by the sample is reported as J. Testing was done at room temperature (23 °C) on as molded samples.

Mold Shrinkage was tested using 101.6 x 3.2 mm molded discs as per an internal method. Both parallel and perpendicular shrinkage measurements were made.

Tensile modulus and tensile elongation at break were tested on injection molded Type 1 bars with a gage length of 50 mm at room temperature with a crosshead speed of 50 mm/min using ASTM D638.

### PBT Polymerization Process

The polymerization reactions in Examples 1-9 were carried out in a 40CV Helicone reactor. The helicone reactor had a capacity of 200 L and was equipped with a special design of twin opposing helical blades with 270 degree twist; constructed of 316 SS with 16 g polish finish. The blade speed could be varied from 1 to 65 rpm. The agitators were connected to a Constant Torque Inverter Duty Motor, which operates at 230/460 VAC, 3 PH, and 60 Hz. These agitators provided excellent surface area for the polymer melt in order to build molecular weight. The helicone was also designed with an overhead condenser to condense the vapors in the transesterification and polymerization stages. The reactor had a conical design with twin rotating blades with close clearance to the reactor walls. This configuration allowed for efficient mixing of high viscosity polymer melts. The 40 CV Helicone reactor was preheated to 170 °C and loaded with all materials. For all batches 45.5 kg of 1,4-butanediol, 65.9 kg of dimethyl terephthalate, and 53.7 mL of tetra(isopropyl) titanate were added. Once the materials were added and the reactor closed, the temperature was ramped from 170°C to 227°C at 2.5 °C/min. During this stage, the 1,4-butanediol reacted with the dimethyl terephthalate and liberated methanol. Both methanol and excess 1,4-butanediol were collected by means of an overhead condenser with cooling water set to 35 °C. Once the rate of overhead collection slowed considerably, vacuum was slowly applied at a target reduction of 33 mbar/min (3.3 kPa/min) while ramping the temperature from 227 °C to 250 °C. The reactor was held at these conditions throughout the polymerization while the molecular weight of the polymer. Additional 1,4-butanediol was collected using the overhead condenser with cooling water at 55 °C. Once the torque in the reactor measured 15 amps, the agitator motor slowed and vacuum seal was broken. Once the agitator stopped, the polymer was discharged from the reactor. The polymer made in this reactor was ground after cooling using a conventional grinder with a mesh screen.

PBT was prepared in Example 10 on an industrial scale through process steps consisting of 2 main steps. First, the polyethylene terephthalate is depolymerized by reaction with ethylene glycol in a process known as glycolysis. The reaction product is then contacted with 1,4-butanediol under conditions suitable for polymerization.

### Dimethyl Terephthalate Compositions

To make the impurity-containing dimethyl terephthalate residue compositions, the amount of dimethyl terephthalate was kept constant and one or more of the following impurities were added in the appropriate ratio: dimethyl isophthalate, cyclohexane dimethanol, diethylene glycol, triethylene glycol.

### Preparation of Stabilized PBT Compositions

Stabilized PBT was prepared in Examples 11-20 by dry mixing 99.74 weight % (wt.%) PBT from Examples 1-10, 0.06 wt.% hindered phenol stabilizer, and 0.2 wt.% hydrocarbon wax release in a paint shaker for approximately 4 min. The mixture was then extruded on a 27 mm Entek Mega Twin Screw Extruder (with a maximum capacity of 34 kg/hr) having 5 feeders and a vacuum vented mixing screw. The feed temperature was 204 °C. The extrusion temperature was maintained between 204-260 °C. The screw speed was 450 rpm. The extrudate was cooled through a water bath prior to pelletizing. Test parts were injection molded on a van Dorn molding machine. The pellets were dried for 4 hr at 121 °C in a forced air-circulating oven prior to injection molding. Parts for testing were molded with a barrel temperature set to 260 °C and the mold temperature set to 66 °C. ASTM parts were made for all tests. Cycle time was approximately 35 s.

### Preparation of Flame Retardant PBT Compositions

A flame retardant PBT with a very high PBT content was prepared in Examples 21-30 by dry mixing 64.46 wt.% PBT from Examples 1-10, 26.0 wt.% brominated polycarbonate, 2.0 wt.% polycarbonate, 6.1 wt.% antimony oxide concentrate, 1.0 wt.% low density polyethylene, 0.06 wt.% hindered phenol stabilizer, 0.08 wt.% SAN encapsulated PTFE-intermediate, and 0.3 wt.% zinc phosphate in a paint shaker for approximately 4 min. The preparation procedure was otherwise identical to that of Examples 11-20.

### Preparation of Impact Modified Polycarbonate/ PBT Compositions

An impact modified polycarbonate / PBT blend using a high PBT content was prepared in Examples 31-40 by dry mixing 66.32 wt.% PBT from Examples 1-10, 15.0 wt.% polycarbonate, 18.0 wt.% MBS rubber, 0.25 wt.% 2-(2'hydroxy-5-t-octylphenyl)-benzotriazole, 0.08 wt.% hindered phenol stabilizer, 0.05 wt.% pentaerythritol beta-lauryl thiopropionate, and 0.3 wt.% anhydrous monosodium phosphate in a paint shaker for approximately 4 min. The preparation procedure was otherwise identical to that of Examples 11-20.

### EXAMPLES 1-10

### Examples 1-4.

The purpose of Example 1 was to prepare PBT from dimethyl terephthalate residue compositions containing dimethyl isophthalate. More specifically, the dimethyl terephthalate composition was made using the procedure described above, except that the following impurity was added: 3.0 wt.% dimethyl isophthalate.

PBT was prepared from dimethyl terephthalate containing 3.0 wt.% dimethyl isophthalate on a pilot scale with the dimethyl terephthalate being composed of 63.9 kg dimethyl terephthalate and 2.0 kg dimethyl isophthalate. The process steps and conditions were otherwise identical to those described above. The results for Example 1 are shown in Table 2.

The purpose of Example 2 was to prepare PBT from dimethyl terephthalate residue compositions containing cyclohexane dimethanol. More specifically, the dimethyl terephthalate composition was made using the procedure described above, except that the following impurities were added: 1.5 wt.% cyclohexane dimethanol.

PBT was prepared from dimethyl terephthalate containing 1.5 wt.% cyclohexane dimethanol on a pilot scale with the dimethyl terephthalate being composed of 64.9 kg of dimethyl terephthalate and 1.0 kg of cyclohexane dimethanol. The process steps and conditions were otherwise identical to those described above. The results for Example 2 are shown in Table 2.

The purpose of Example 3 was to prepare PBT from dimethyl terephthalate residue compositions containing diethylene glycol and triethylene glycol. More specifically, the dimethyl terephthalate composition was made using the procedure described above, except that the following impurities were added: 0.5 wt.% diethylene glycol and 0.1 wt.% triethylene glycol.

PBT was prepared from dimethyl terephthalate containing 0.5 wt.% diethylene glycol and 0.1 wt.% triethylene glycol on a pilot scale with the dimethyl terephthalate being composed of 65.5 kg of dimethyl terephthalate, 330 g of diethylene glycol and 66 g of triethylene glycol. The process steps and conditions were otherwise identical to those described above. The results for Example 3 are shown in Table 2.

The purpose of Example 4 was to prepare PBT from dimethyl terephthalate residue compositions containing dimethyl isophthalate, cyclohexane dimethanol, diethylene glycol, and triethylene glycol. More specifically, the dimethyl terephthalate composition was made using the procedure described above, except that the following impurities were added: 3 wt.% dimethyl isophthalate, 1.5 wt.% cyclohexane dimethanol, 0.5 wt.% diethylene glycol, and 0.1 wt.% triethylene glycol.

PBT was prepared from dimethyl terephthalate containing 3 wt.% dimethyl isophthalate, 1.5 wt.% cyclohexane dimethanol, 0.5 wt.% diethylene glycol, and 0.1 wt.% triethylene glycol on a pilot scale with the dimethyl terephthalate being composed of 62.7 kg of dimethyl terephthalate, 2.0 kg of dimethyl isophthalate, 1.0 kg of cyclohexane dimethanol, 330 g of diethylene glycol and 66 g of triethylene glycol. The process steps and conditions were otherwise identical to those described above. The results for Example 4 are shown in Table 2.

| Ex. | DMI | CHDM | DEG | TEG | DMT Residual Composition Residue Level | Onset Tₘ (°C) | Onset T_{c} (°C) | L* | a* | b* |
|---|---|---|---|---|---|---|---|---|---|---|
| 1 | 3.0 | 0 | 0 | 0 | 3.0 | 220.6 | 178.0 | 83.91 | 0.849 | 8.41 |
| 2 | 0 | 1.5 | 0 | 0 | 1.5 | 222.1 | 177.8 | 84.31 | 0.702 | 7.93 |
| 3 | 0 | 0 | 0.5 | 0.1 | 0.6 | 224.2 | 177.8 | 85.87 | 0.157 | 5.47 |
| 4 | 3.0 | 1.5 | 0.5 | 0.1 | 5.1 | 216.3 | 168.4 | 86.48 | 0.48 | 6.62 |
| 5 | 0 | 0 | 0 | 0 | 0 | 225.4 | 180.5 | 85.98 | 1.113 | 9.35 |
| 6 | 0 | 0 | 0 | 0 | 0 | 223.9 | 186.9 | 86.69 | 0.509 | 6.44 |
| 7 | 0 | 0 | 0 | 0 | 0 | 225.5 | 177.6 | 89.56 | -0.039 | 3.77 |
| 8 | 0 | 0 | 0 | 0 | 0 | 225.8 | 176.3 | 87.54 | -0.094 | 3.64 |
| 9 | 0 | 0 | 0 | 0 | 0 | 225.4 | 182.2 | 87.36 | -0.201 | 4.15 |
| 10 | n/d | n/d | n/d | n/d | 4.1 | 215.7 | 174.9 | 73.64 | -2.025 | 2.77 |

### Comparative Examples 5-9.

The purpose of Comparative Examples 5-9 was to prepare PBT from dimethyl terephthalate using the procedure described above, but with no impurities added in the dimethyl terephthalate.

PBT was prepared on the pilot scale using 65.0 kg of dimethyl terephthalate. The process steps and testing conditions were otherwise identical to those described above for Examples 1-4. The results for Comparative Examples 5-9 are shown in Table 2. Comparative Example 10.

The purpose of Comparative Example 10 was to compare PBT made from polyethylene terephthalate. This material is used for comparison purposes with Examples 1-4. While this material is not made from dimethyl terephthalate, this PBT contains a known amount of impurities that are ordinarily present in recycled polyethylene terephthalate sources. The results for Comparative Example 10 are shown in Table 2.

Table 2 shows the measured properties for Examples 1-4 and Comparative Examples 5-10. Amounts are shown in weight percent (wt.%).
Table 2.

The results in Table 2 show that the use of only certain dimethyl terephthalate compositions containing specific levels of impurities produced polybutylene terephthalate having suitable properties.

PBT polymers made in Comparative Examples 5-9 from dimethyl terephthalate were each tested twice. This allowed establishment of total process variation, including testing. This baseline was then used to detect any significant deviation in polymer properties of the materials made in Examples 1-4 from the Comparative Examples. A significant deviation was a property measurement more than 2 standard deviations from the baseline properties established from the Comparative Examples.

The color of polymers made from recycled materials is often an issue. The L*, a*, b* measurements for the produced polymers are given in Table 3. Comparative Example 10 gives results that are typical of PBT made from recycled polyethylene terephthalate sources by glycolysis. The color measurements for Examples 1-4 are all within the normal process variation. Presence of the co-monomers in the amounts used in Examples 1-4 does not have a significant adverse effect on the color of the produced PBT.

**Table 3. Comparison polymer color of Examples 1-4 to the baseline PBT.**

| Ex. | Co-monomers | L* | a* | b* |
|---|---|---|---|---|
| 1 | 3% DMI | 84 | 0.8 | 8.4 |
| 2 | 1.5% CHDM | 84 | 0.7 | 7.9 |
| 3 | 0.5% DEG, 0.1% TEG | 86 | 0.2 | 5.5 |
| 4 | 3% DMI, 1.5% CHDM, 0.5% DEG, 0.1% TEG | 86 | 0.5 | 6.6 |
| 5-9 | None | 84 - 91 | -0.9 - 1.3 | 0.9 - 9.3 |
| 10 | 4.1% Total | 74 | -2.0 | 2.8 |

In the pilot scale helicone reactor, the molecular weight of the polymer was not tightly controlled. Any dependence of melt viscosity, melt volume rate, and intrinsic viscosity on the impurities in dimethyl terephthalate was insignificant compared to the molecular weight variations inherent to the process.

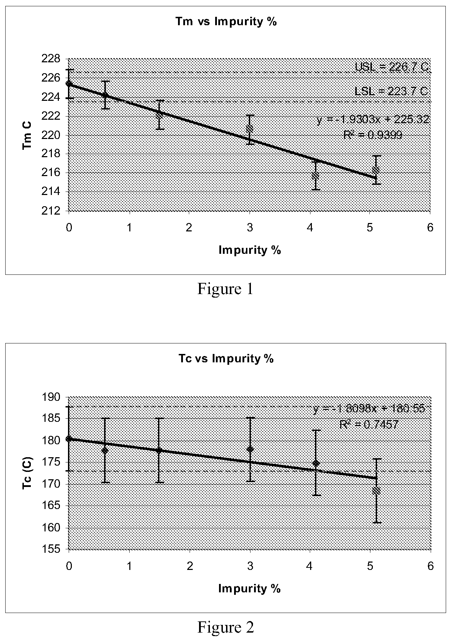

The melting point (Tₘ) and the crystallization temperature (T_{c}) of the polymers made in Examples 1-10 as a function of dimethyl terephthalate impurity are shown in Figure 1 and Figure 2, respectively. The points at 0 wt.% impurity correspond to the average of the 5 batches of PBT made from virgin dimethyl terephthalate in Examples 5-9, each measured twice. The standard deviation for the process was calculated from the PBT made from virgin dimethyl terephthalate. The error bars on the points are given as 2 process standard deviations on each side. The specifications shown in the Figures correspond to 2 standard deviations on either side of the standard PBT. These are included to see if a significant change in the measured property occurs due to the impurities in the dimethyl terephthalate. Points marked with squaresindicate measurements that are significantly different than the baseline established using Examples 5-9. Both Tₘ and T_{c} show a dependence upon the amount of impurities in the dimethyl terephthalate used to make PBT. The effect of impurities on Tₘ is much stronger than their effect on T_{c} relative to the process standard deviation and has a linear relationship between the Tₘ and the impurity level. From the thermal data, it does not appear that the type of impurity has an impact, only the total amount of impurity in the dimethyl terephthalate. Surprisingly, even very low impurity levels result in significant changes to the Tₘ of the polymer. Surprisingly, if the total amount of impurities present in the dimethyl terephthalate is more than only 0.8 wt.%, the PBT will have a significantly different melting point. To ensure that the PBT made from recycled dimethyl terephthalate has equivalent melting point properties, the recycled dimethyl terephthalate must contain less than 0.8 wt.% of the impurities.

### EXAMPLES 11-20

### Examples 11-14.

The purpose of Example 11 was to prepare stabilized PBT from PBT made from dimethyl terephthalate residue compositions containing dimethyl isophthalate. More specifically, the stabilized PBT composition was prepared using PBT made from dimethyl terephthalate residue compositions containing 3.0 wt.% dimethyl isophthalate from Example 1, hindered phenol stabilizer, and hydrocarbon wax release according to the procedure described above. The testing procedures were identical to those described above. The results for Example 11 are shown in Table 4.

The purpose of Example 12 was to prepare stabilized PBT from PBT made from dimethyl terephthalate residue compositions containing cyclohexane dimethanol. More specifically, the stabilized PBT composition was prepared using PBT made from dimethyl terephthalate residue compositions containing 1.5 wt.% cyclohexane dimethanol from Example 2, hindered phenol stabilizer, and hydrocarbon wax release according to the procedure described above. The process steps and conditions were otherwise identical to those described above. The results for Example 12 are shown in Table 4.

The purpose of Example 13 was to prepare stabilized PBT from PBT made from dimethyl terephthalate residue compositions containing diethylene glycol and triethylene glycol. More specifically, the stabilized PBT composition was prepared using PBT prepared using dimethyl terephthalate residue compositions containing 0.5 wt.% diethylene glycol and 0.1 wt.% triethylene glycol from Example 3, hindered phenol stabilizer, and hydrocarbon wax release according to the procedure described above. The process steps and conditions were otherwise identical to those described above. The results for Example 13 are shown in Table 4.

The purpose of Example 14 was to prepare stabilized PBT from PBT made from dimethyl terephthalate residue compositions containing dimethyl isophthalate, cyclohexane dimethanol, diethylene glycol, and triethylene glycol. More specifically, the stabilized PBT composition was prepared using PBT prepared from dimethyl terephthalate residue compositions containing 3 wt.% dimethyl isophthalate, 1.5 wt.% cyclohexane dimethanol, 0.5 wt.% diethylene glycol and 0.1 wt.% triethylene glycol from Example 4, hindered phenol stabilizer, and hydrocarbon wax release. The process steps and conditions were otherwise identical to those described above. The results for Ex. 14 are in Table 4. Comparative Examples 15-19.

The purpose of Comparative Examples 15-19 was to establish baseline process variation for the production of stabilized PBT compositions made from virgin dimethyl terephthalate, a material that contained substantially less of the impurities that were found in the dimethyl terephthalate residue compositions. The stabilized PBT was prepared from PBT made from the dimethyl terephthalate residues without added impurities in Comparative Examples 5-9, hindered phenol stabilizer, and hydrocarbon wax release according to the procedure described above. The process steps and conditions were otherwise identical to those described above. The results for Comparative Examples 15-19 are shown in Table 4. Comparative Example 20.

The purpose of Comparative Example 20 was to compare stabilized PBT compositions comprising PBT made from recycled polyethylene terephthalate. This material is used for comparison purposes with Examples 11-14. While this material is not made from dimethyl terephthalate, this stabilized PBT contains a known amount of impurities that are ordinarily present in recycled polyethylene terephthalate sources. The stabilized PBT was prepared using PBT made from polyethylene terephthalate from Comparative Example 10, hindered phenol stabilizer, and hydrocarbon wax release according to the procedure described above. The process steps and conditions were otherwise identical to those described above. The results for Comparative Example 20 are shown in Table 4.

Table 4 shows the results for stabilized PBT made in Examples 11-20.

**Table 4.**

| Ex. | DMT Residual Level | FM (MPa) | 0.455 MPa HDT (°C) | 1.82 MPa HDT (°C) | NIIS (J/m) | UIIS (J/m) | Total Energy (J) | MS P11 (%) | MS Ppn (%) | E (MPa) | ε_{y} (%) |
|---|---|---|---|---|---|---|---|---|---|---|---|
| 11 | 3.0 | 2520 | 113.5 | 46.8 | 49.6 | 2130 | 56.1 | 1.97 | 1.52 | 2770 | 190.8 |
| 12 | 1.5 | 2510 | 141 | 49.8 | 49 | 2100 | 51.7 | 1.96 | 1.59 | 2690 | 246.4 |
| 13 | 0.6 | 2540 | 145.7 | 49.3 | 49.5 | 2130 | 64.8 | 2.06 | 2.08 | 2770 | 96.1 |
| 14 | 5.1 | 2470 | 120.9 | 46.2 | 51 | 2090 | 45.9 | 1.94 | 1.85 | 2690 | 283.6 |
| 15 | 0 | 2590 | 155 | 49.9 | 49.5 | 1750 | 29.4 | 2.27 | 2.23 | 2850 | 6.9 |
| 16 | 0 | 2570 | 151.8 | 49.1 | 28.3 | 2040 | 12.8 | 2.08 | 2.15 | 2810 | 68.8 |
| 17 | 0 | 2480 | 146.8 | 47.7 | 37.8 | 1830 | 41.5 | 2.17 | 2.2 | 2720 | 123.5 |
| 18 | 0 | 2580 | 153.3 | 52.1 | 27.2 | 572 | 4.54 | 2.16 | 2.15 | 2810 | 5.3 |
| 19 | 0 | 2490 | 148.5 | 48.9 | 26.4 | 2140 | 77.8 | 2.11 | 2.18 | 2650 | 89.8 |
| 20 | 4.1 | 2540 | 115.2 | 46.6 | 51.9 | 2140 | 4.503 | 2.02 | 2.04 | 2730 | 276 |

| | | | | | | | | | | | |
|---|---|---|---|---|---|---|---|---|---|---|---|
| FM -flexural modulus UIIS - Unnotched Izod impact strength MSP11 - Mold shrinkage in the parallel direction, MSPpn - Mold shrinkage in the perpendicular direction NIIS- Notched Izod impact strength, E - modulus of elasticity ε_{y} - elongation at break | | | | | | | | | | | |

Stabilized PBT polymers made in Comparative Examples 15-19 containing PBT produced from dimethyl terephthalate were each extruded, molded, and tested twice. This allowed establishment of total process variation, including testing. This baseline was then used to detect any significant deviation in polymer properties of the materials made in Examples 11-14 from the Comparative Examples 15-19. A significant deviation was a property measurement more than 2 standard deviations from the baseline properties established from the Comparative Examples 15-19.

Measurements of flexural modulus, notched Izod impact strength, un-notched Izod impact strength, and the modulus of elasticity for Examples 11-20 are all within the normal process variation. The presence of the co-monomers in the amounts used in Examples 1-4 does not have a significant adverse effect on these properties of the stabilized PBT compositions.

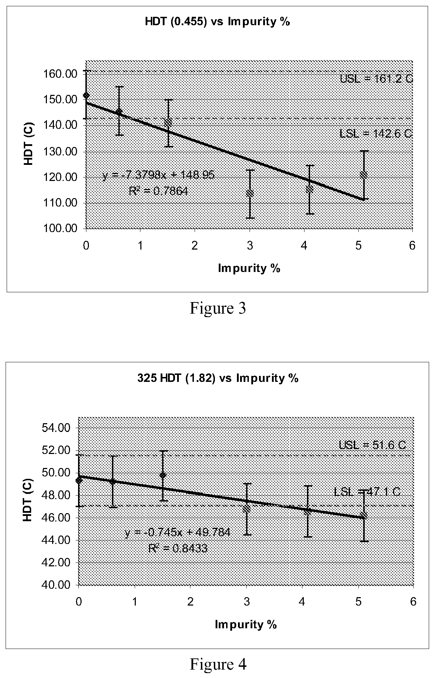

The heat deflection temperature measurements of the stabilized PBT polymers with a loading stress of 0.455 MPa and 1.82 MPa are shown in Figure 3 and Figure 4, respectively. Points marked with squares indicate measurements that are significantly different than the baseline established using Examples 15-19. The heat deflection temperature measurements for Examples 11-14 show a significant reduction when impurities are present in the dimethyl terephthalate used to make the stabilized PBT. Surprisingly, the presence of the co-monomers in the amounts used in Examples 1-4 does have a significant adverse effect on the heat deflection temperature of the produced stabilized PBT. This is believed to be due to incorporation of the impurities as co-monomers in the polymer backbone resulting in a decrease in the crystallinity of the polymer. Surprisingly, even low impurity levels result in significant changes to the heat deflection temperature of the polymer. If the total impurities present in the dimethyl terephthalate are more than only 0.9 wt.%, the stabilized PBT may have a significantly different heat deflection temperature. To ensure that the stabilized PBT made from recycled dimethyl terephthalate has an equivalent heat deflection temperature, the recycled dimethyl terephthalate must contain less than 0.9 wt.% of impurities.

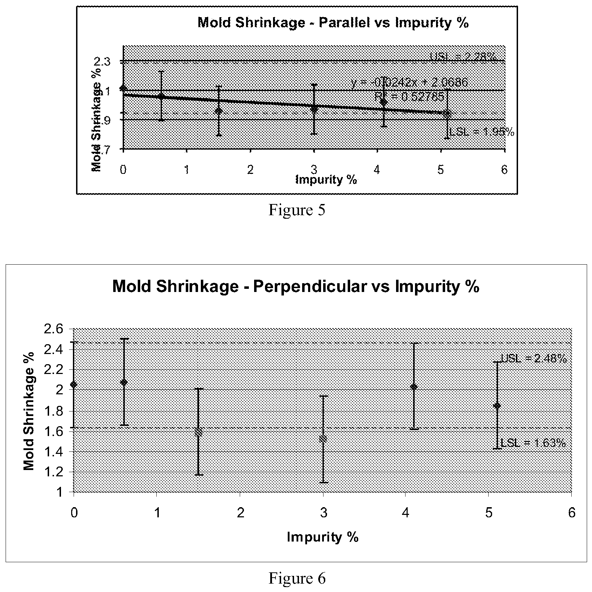

The mold shrinkage measurements in the parallel direction and in the perpendicular direction of the stabilized PBT compositions are shown in Figure 5 and Figure 6, respectively. Both the mold shrinkage measurements in the parallel direction and in the perpendicular direction show a dependence upon the amount of impurities in the dimethyl terephthalate used to make PBT. Surprisingly, even low impurity levels result in significant changes to the mold shrinkage of the polymer. This is also believed to be due to incorporation of the impurities as co-monomers in the polymer backbone resulting in a decrease in the crystallinity of the polymer. If the total impurities present in the dimethyl terephthalate are more than only 5.1 wt.% the stabilized PBT may have significantly different mold shrinkage in the parallel direction and if the total impurities present in the dimethyl terephthalate are more than only 1.5 wt.% the stabilized PBT may have significantly different mold shrinkage in the perpendicular direction. To ensure that the stabilized PBT made from recycled dimethyl terephthalate has equivalent mold shrinkage, the recycled dimethyl terephthalate must contain less than 1.5 wt.% of impurities.

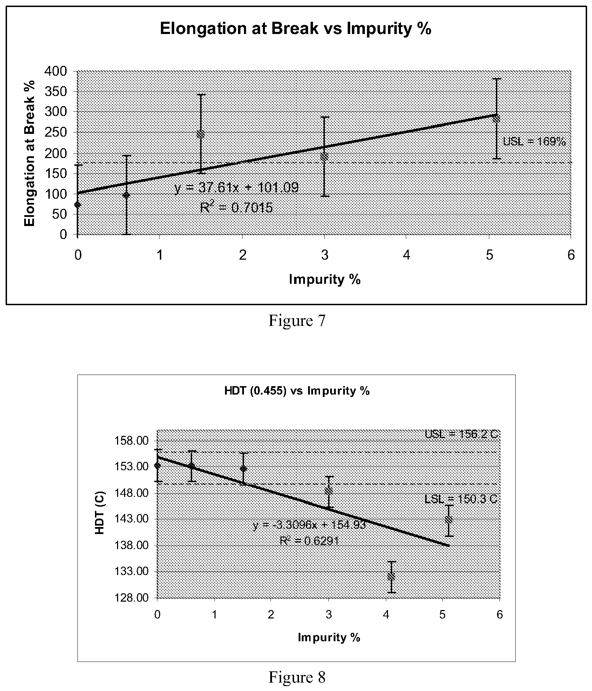

The tensile elongation at break measurements of the stabilized PBT compositions are shown in Figure 7. The tensile elongation at break measurements show a dependence upon the amount of impurities in the dimethyl terephthalate used to make PBT. Surprisingly, even low impurity levels result in significant changes to the elongation at break of the polymer. If the total impurities present in the dimethyl terephthalate are more than only 1.5 wt.%, the stabilized PBT may have significantly different elongation at break. To ensure that the stabilized PBT made from recycled dimethyl terephthalate has equivalent elongation at break, the recycled dimethyl terephthalate must contain less than 1.5 wt.% of impurities.

To ensure that the stabilized PBT made from dimethyl terephthalate residual compositions has equivalent heat deflection properties, tensile elongation at break, and mold shrinkage in the perpendicular and parallel directions, as well as flexural modulus, notched Izod impact strength, un-notched Izod impact strength, mold shrinkage in both the parallel and perpendicular directions, the modulus of elasticity, and elongation at break, the recycled dimethyl terephthalate must contain less than 0.9 wt.% of impurities.

### EXAMPLES 21-30

### Examples 21-24

The purpose of Example 21 was to prepare a flame retardant PBT with a very high PBT content made from dimethyl terephthalate residue compositions. More specifically, the flame retardant was prepared using PBT made from dimethyl terephthalate residue compositions containing 3.0 wt.% dimethyl isophthalate from Example 1, brominated polycarbonate, polycarbonate, hindered phenol stabilizer, antimony oxide concentrate, SAN encapsulated PTFE-intermediate, low density polyethylene, and zinc phosphate according to the procedure described above. The process steps and conditions were otherwise identical to those described above. The results for Example 21 are shown in Table 5.

The purpose of Example 22 was to prepare a flame retardant PBT with a very high PBT content from PBT made from dimethyl terephthalate residue compositions containing cyclohexane dimethanol. More specifically, the flame retardant was prepared using PBT made from dimethyl terephthalate residue compositions containing 1.5 wt.% cyclohexane dimethanol from Example 2, brominated polycarbonate, polycarbonate, hindered phenol stabilizer, antimony oxide concentrate, SAN encapsulated PTFE-intermediate, low density polyethylene, and zinc phosphate according to the procedure described above. The process steps and conditions were otherwise identical to those described above. The results for Example 22 are shown in Table 5.

The purpose of Example 23 was to prepare a flame retardant PBT with a very high PBT content from PBT made from dimethyl terephthalate residue compositions containing diethylene glycol and triethylene glycol. More specifically, the flame retardant was prepared using PBT made from dimethyl terephthalate residue compositions containing 0.5 wt.% diethylene glycol and 0.1 wt.% triethylene glycol from Example 3, brominated polycarbonate, polycarbonate, hindered phenol stabilizer, antimony oxide concentrate, SAN encapsulated PTFE-intermediate, low density polyethylene, and zinc phosphate according to the procedure described above. The process steps and conditions were otherwise identical to those described above. The results for Example 23 are shown in Table 5.

The purpose of Example 24 was to prepare a flame retardant with a very high PBT content from PBT made from dimethyl terephthalate residue compositions containing dimethyl isophthalate, cyclohexane dimethanol, diethylene glycol, and triethylene glycol. More specifically, the flame retardant was prepared using PBT made from dimethyl terephthalate residue compositions containing 3.0 wt.% dimethyl isophthalate, 1.5 wt.% cyclohexane dimethanol, 0.5 wt.% diethylene glycol and 0.1 wt.% triethylene glycol from Example 4, brominated polycarbonate, polycarbonate, hindered phenol stabilizer, antimony oxide concentrate, SAN encapsulated PTFE-intermediate, low density polyethylene, and zinc phosphate according to the procedure described above. The process steps and conditions were otherwise identical to those described above. The results for Example 24 are shown in Table 5.

### Comparative Examples 25-29

The purpose of Comparative Examples 25-29 was to establish baseline process variation for the production of a flame retardant with a very high PBT content produced from the polymer made from virgin dimethyl terephthalate, a material that contained substantially less of the impurities that were found in the dimethyl terephthalate residue compositions. This material is used for comparison with Examples 21-24. The flame retardant PBT was prepared using PBT made from dimethyl terephthalate from Comparative Examples 5-9, brominated polycarbonate, polycarbonate, hindered phenol stabilizer, antimony oxide concentrate, SAN encapsulated PTFE-intermediate, low density polyethylene, and zinc phosphate according to the procedure described above. PBT is made from virgin dimethyl terephthalate using the same processing and testing conditions as used in Examples 21-24. The results for Comparative Examples 25-29 are shown in Table 5.

### Comparative Example 30

The purpose of Example 30 was to compare a flame retardant with a very high PBT content made from PBT made from polyethylene terephthalate. This material is used for comparison purposes with Examples 21-24. While this material is not made from dimethyl terephthalate, this flame retardant with a very high PBT content contains a known amount of impurities that are ordinarily present in recycled polyethylene terephthalate sources. The flame retardant PBT was prepared using polyethylene terephthalate from comparative Example 10, brominated polycarbonate, polycarbonate, hindered phenol stabilizer, antimony oxide concentrate, SAN encapsulated PTFE-intermediate, low density polyethylene, and zinc phosphate according to the procedure described above. The testing procedures were identical to those described above. The results for Comparative Example 30 are shown in Table 5.

**Table 5.**

| Ex. | DMT Residual Composition Level | FM (MPa) | 0.455 MPa HDT (°C) | 1.82 MPa HDT (°C) | NIIS (J/m) | UIIS (J/m) | Total Energy (J) | MSP11 (%) | MSPpn (%) | E (MPa) | ε_{y} (%) |
|---|---|---|---|---|---|---|---|---|---|---|---|
| 21 | 3.0 | 2710 | 148.3 | 66.1 | 53.4 | 1470 | 23.4 | 1.6 | 1.6 | 2920 | 11.1 |
| 22 | 1.5 | 2690 | 152.7 | 68.1 | 49.6 | 1290 | 8.86 | 1.55 | 1.55 | 2870 | 4.9 |
| 23 | 0.6 | 2720 | 153.2 | 75.2 | 51 | 1490 | 51.6 | 1.58 | 1.58 | 2920 | 8.7 |
| 24 | 5.1 | 2640 | 142.8 | 60.4 | 54.8 | 1630 | 7.66 | 1.49 | 1.43 | 2830 | 6.6 |
| 25 | 0 | 2710 | 154.5 | 69.8 | 51.9 | 1520 | 22.1 | 1.62 | 1.61 | 2910 | 5.4 |
| 26 | 0 | 2670 | 153.4 | 68.8 | 30.4 | 1810 | 29.3 | 1.81 | 1.85 | 2830 | 10.3 |
| 27 | 0 | 2680 | 154.4 | 70.2 | 33.1 | 1470 | 36.7 | 1.82 | 1.84 | 2830 | 9.5 |
| 28 | 0 | 2700 | 154.9 | 70.1 | 30.8 | 1920 | 40.5 | 1.88 | 1.9 | 2850 | 14 |
| 29 | 0 | 2660 | 152.4 | 65.1 | 30.8 | 1770 | 54 | 1.81 | 1.83 | 2820 | 15.2 |
| 30 | 4.1 | 2710 | 132 | 61.2 | 55.9 | 2130 | 63.5 | 1.38 | 1.37 | 2900 | 24.9 |

| | | | | | | | | | | | |
|---|---|---|---|---|---|---|---|---|---|---|---|
| M -flexural modulus UIIS - Unnotched Izod impact strength MSP11 - Mold shrinkage in the parallel direction, MSPpn - Mold shrinkage in the perpendicular direction NIIS- Notched Izod impact strength, E - modulus of elasticity ε_{y} - elongation at break | | | | | | | | | | | |

Flame retardant PBT compositions of Comparative Examples 25-29 made from PBT from dimethyl terephthalate were each extruded, molded, and tested twice. This allowed establishment of total process variation, including testing. This baseline was then used to detect any significant deviation in properties of the materials made in Examples 21-24 from the Comparative Examples 25-29. A significant deviation was a property measurement more than 2 standard deviations from the baseline properties established from the Comparative Examples 25-29. The points at 0 wt.% impurity correspond to the average of the 5 batches using PBT made from virgin dimethyl terephthalate in Examples 25-29, each measured twice. Points marked with squares indicate measurements that are significantly different than the baseline established using Examples 25-29.

Measurements of the flexural modulus, notched Izod impact strength, un-notched Izod impact strength, mold shrinkage in the parallel and perpendicular directions, modulus of elasticity, and tensile elongation at break of the produced flame retardant with a very high PBT content do not show any correlation that depends on the amount of impurities present. Presence of the co-monomers in the amounts used in Examples 1-4 does not have a significant adverse effect on these properties of the produced flame retardant with a very high PBT content.

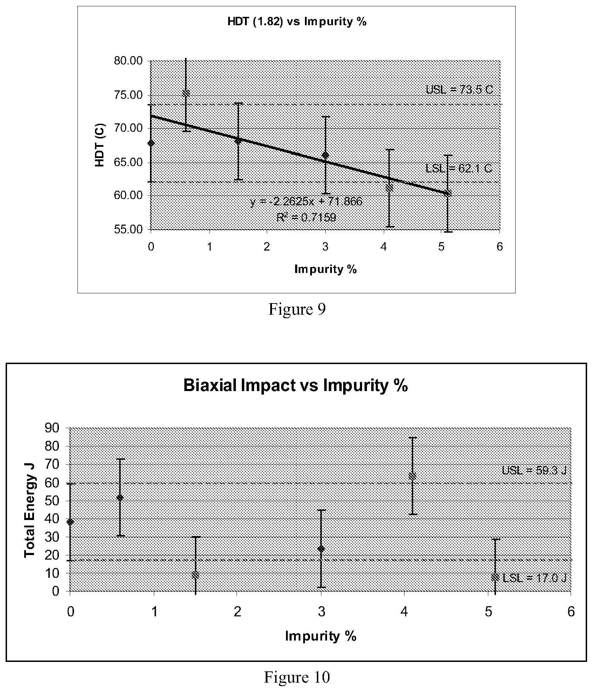

The heat deflection temperature measurements of the produced flame retardant with a very high PBT content with a loading stress of 0.455 MPa and 1.82 MPa are given in Figure 8 and Figure 9, respectively. The heat deflection temperature measurements for Examples 21-24 show a significant reduction when impurities are present in the dimethyl terephthalate used to make the flame retardant with a very high PBT content. Surprisingly, the presence of the co-monomers in the amounts used in Examples 1-4 does have a significant adverse effect on the heat deflection temperature of the produced flame retardant with a very high PBT content. This is believed to be due to incorporation of the impurities as co-monomers in the PBT backbone resulting in a decrease in the crystallinity of the polymer. Surprisingly, even low impurity levels result in significant changes to the heat deflection temperature of the flame retardant with a very high PBT content. If the total impurities present in the dimethyl terephthalate are more than only 1.4 wt.%, the flame retardant with a very high PBT content may have a significantly different heat deflection temperature. To ensure that the flame retardant with a very high PBT content made from recycled dimethyl terephthalate has an equivalent heat deflection temperature, the recycled dimethyl terephthalate must contain less than 1.4 wt.% of impurities.

The total energy measurements from the biaxial impact test of the produced flame retardant with a very high PBT content are given in Figure 10. The total energy measurements for Examples 21-24 do not have a correlation to the amount of impurities present in the dimethyl terephthalate used to make the PBT. However, three of the examples do have total energy measurements from the biaxial impact test that are significantly different than the Comparative Examples. If the total impurities present in the dimethyl terephthalate are more than only 1.5 wt.%, the flame retardant with a very high PBT content may have a significantly different total energy measured by the biaxial impact test. To ensure that the flame retardant with a very high PBT content made from recycled dimethyl terephthalate has equivalent properties, the recycled dimethyl terephthalate must contain less than 1.5 wt.% of impurities

### EXAMPLES 31-40

### Examples 31-34

The purpose of Example 31 was to prepare an impact modified polycarbonate / PBT blend using a high PBT content from PBT made from dimethyl terephthalate residue compositions containing dimethyl isophthalate. More specifically, the impact modified polycarbonate / PBT blend was prepared using PBT made from dimethyl terephthalate residue compositions containing 3.0 wt.% dimethyl isophthalate from Example 1, polycarbonate, MBS, 2-(2'hydroxy-5-t-octylphenyl)-benzotriazole, hindered phenol stabilizer, pentaerythritol beta-lauryl thiopropionate, and anhydrous monosodium phosphate according to the procedure described above. The process steps and conditions were otherwise identical to those described above. The results for Example 31 are in Table 6.

The purpose of Example 32 was to prepare an impact modified polycarbonate / PBT blend using a high PBT content from PBT made from dimethyl terephthalate residue compositions containing cyclohexane dimethanol. More specifically, the impact modified polycarbonate / PBT blend was prepared using PBT made from dimethyl terephthalate residue compositions containing 1.5 wt.% cyclohexane dimethanol from Example 2, polycarbonate, MBS, 2-(2'hydroxy-5-t-octylphenyl)-benzotriazole, hindered phenol stabilizer, pentaerythritol beta-lauryl thiopropionate, and anhydrous monosodium phosphate according to the procedure described above. The process steps and conditions were otherwise identical to those described above. The results for Example 32 are shown in Table 6.

The purpose of Example 33 was to prepare an impact modified polycarbonate / PBT blend using a high PBT content from PBT made from dimethyl terephthalate residue compositions containing diethylene glycol and triethylene glycol. More specifically, the impact modified polycarbonate / PBT blend was prepared using PBT made from dimethyl terephthalate residue compositions containing 0.5 wt.% diethylene glycol and 0.1 wt.% triethylene glycol from Example 3, polycarbonate, MBS rubber, 2-(2'hydroxy-5-t-octylphenyl)-benzotriazole, hindered phenol stabilizer, pentaerythritol beta-lauryl thiopropionate, and anhydrous monosodium phosphate according to the procedure described above. The process steps and conditions were otherwise identical to those described above. The results for Example 33 are shown in Table 6.

The purpose of Example 34 was to prepare an impact modified polycarbonate/PBT blend using a high PBT content from PBT made from dimethyl terephthalate residue compositions containing dimethyl isophthalate, cyclohexane dimethanol, diethylene glycol, and triethylene glycol. More specifically, the impact modified polycarbonate / PBT blend was prepared using PBT made from dimethyl terephthalate residue compositions containing 3.0 wt.% dimethyl isophthalate, 1.5 wt.% cyclohexane dimethanol, 0.5 wt.% diethylene glycol and 0.1 wt.% triethylene glycol from Example 4, polycarbonate, MBS rubber, 2-(2'hydroxy-5-t-octylphenyl)-benzotriazole, hindered phenol stabilizer, pentaerythritol beta-lauryl thiopropionate, and anhydrous monosodium phosphate according to the procedure described above. The process steps and conditions were otherwise identical to those described above. The results for Example 34 are in Table 6. Comparative Examples 35-39

The purpose of Comparative Examples 35-39 was to establish baseline process variation for the production of an impact modified with a polycarbonate / PBT blend using a high PBT content produced from the polymer made from virgin dimethyl terephthalate, a material that contained substantially less of the impurities that were found in the dimethyl terephthalate residue compositions. The impact modified with a polycarbonate/PBT blend was prepared using a high PBT content from PBT made from dimethyl terephthalate from Comparative Examples 5-9, polycarbonate, MBS rubber, 2-(2'hydroxy-5-t-octylphenyl)-benzotriazole, hindered phenol stabilizer, pentaerythritol beta-lauryl thiopropionate, and anhydrous monosodium phosphate according to the procedure described above. The process steps and conditions were otherwise identical to those described above. This material is used for comparison purposes with Examples 31-34. The results for Comparative Examples 35-39 are shown in Table 6.

### Comparative Example 40

The purpose of Comparative Example 40 was to compare an impact modified polycarbonate / PBT blend using a high PBT content from PBT made from polyethylene terephthalate. This material is used for comparison purposes with Examples 31-34. While this material is not made from dimethyl terephthalate, this impact modified with a polycarbonate / PBT blend using a high PBT content contains a known amount of impurities that are ordinarily present in recycled polyethylene terephthalate sources. The impact modified with a polycarbonate / PBT blend was prepared using a high PBT content from PBT made from polyethylene terephthalate from Comparative Example 10, polycarbonate, MBS rubber, 2-(2'hydroxy-5-t-octylphenyl)-benzotriazole, hindered phenol stabilizer, pentaerythritol beta-lauryl thiopropionate, and anhydrous monosodium phosphate according to the procedure described above. The testing procedures were identical to those described above. The results for Comparative Example 40 are shown in Table 6.

Table 6 shows the properties for an impact modified polycarbonate / PBT blend using a high PBT content of Examples 31-40.

**Table 6.**

| Ex. | DMT Residual Composition Level | FM (MPa) | 0.455 MPa HDT (°C) | 1.82 MPa HDT (°C) | NIIS (J/m) | UIIS (J/m) | Total Energy (J) | MSP11 (%) | MSPpn (%) | E (MPa) | ε_{y} (%) |
|---|---|---|---|---|---|---|---|---|---|---|---|
| 31 | 3.0 | 1840 | 93.6 | 52.1 | 786 | 2040 | 52.7 | 1.44 | 1.43 | 1790 | 147.6 |
| 32 | 1.5 | 1830 | 96.4 | 53.3 | 755 | 1940 | 53.3 | 1.44 | 1.42 | 1750 | 71.6 |
| 33 | 0.6 | 1800 | 95.6 | 53.3 | 791 | 1970 | 56.5 | 1.45 | 1.44 | 1780 | 96.2 |
| 34 | 5.1 | 1780 | 82.5 | 51.4 | 858 | 1870 | 54.6 | 1.45 | 1.47 | 1710 | 155.1 |
| 35 | 0 | 1860 | 100.6 | 55.2 | 810 | 2040 | 54.6 | 1.52 | 1.5 | 1810 | 87.2 |
| 36 | 0 | 1770 | 95.2 | 52.8 | 838 | 1920 | 56.4 | 1.43 | 1.44 | 1780 | 178.9 |
| 37 | 0 | 1770 | 95.9 | 52.8 | 872 | 2050 | 56.2 | 1.51 | 1.53 | 1750 | 235.3 |
| 38 | 0 | 1740 | 93.8 | 52.7 | 823 | 1920 | 53.1 | 1.46 | 1.53 | 1730 | 103.4 |
| 39 | 0 | 1760 | 91.2 | 53.9 | 836 | 2040 | 56.3 | 1.45 | 1.45 | 1780 | 126.9 |
| 40 | 4.1 | 1760 | 87.8 | 51.9 | 842 | 2000 | 55.6 | 1.37 | 1.35 | 1660 | 236.2 |

| | | | | | | | | | | | |
|---|---|---|---|---|---|---|---|---|---|---|---|
| FM -flexural modulus UIIS - Unnotched Izod impact strength MSP11 - Mold shrinkage in the parallel direction, MSPpn - Mold shrinkage in the perpendicular direction NIIS- Notched Izod impact strength, E - modulus of elasticity ε_{y} - elongation at break | | | | | | | | | | | |

Impact modified polycarbonate / PBT polymers made using a high PBT content made in Comparative Examples 35-39 made from PBT from dimethyl terephthalate were each extruded, molded, and tested twice. This allowed establishment of total process variation, including testing. This baseline was then used to detect any significant deviation in properties of the materials made in Examples 31-34 from the Comparative Examples 35-39. A significant deviation was a property measurement more than 2 standard deviations from the baseline properties established from the Comparative Examples 35-39. The points at 0 wt.% impurity correspond to the average of the 5 batches using PBT made from virgin dimethyl terephthalate in Examples 35-39, each measured twice. Points marked with squares indicate measurements that are significantly different than the baseline established using Examples 35-39.

Measurements of the flexural modulus, notched Izod impact strength, un-notched Izod impact strength, total energy measured by the biaxial impact test, mold shrinkage in the parallel and perpendicular directions, modulus of elasticity, and tensile elongation at break of the produced impact modified with a polycarbonate / PBT blend using a high PBT content do not show any correlation that depends on the amount of impurities present. Presence of the co-monomers in the amounts used in Examples 1-4 does not have a significant adverse effect on these properties of the produced impact modified with a polycarbonate / PBT blend using a high PBT content.

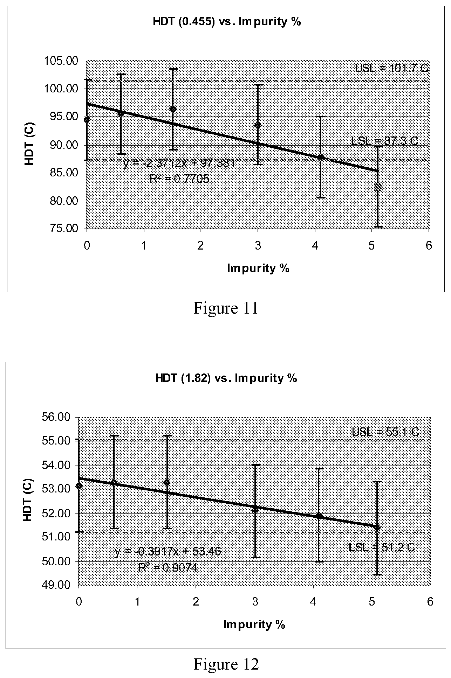

The heat deflection temperature measurements of the produced impact modified with a polycarbonate / PBT blend using a high PBT content with a loading stress of 0.455 MPa and 1.82 MPa are given in Figure 11 and Figure 12, respectively. The heat deflection temperature measurements for Examples 31-34 show a significant reduction when impurities are present in the dimethyl terephthalate used to make the impact modified with a polycarbonate / PBT blend using a high PBT content. Surprisingly, the presence of the co-monomers in the amounts used in Examples 1-4 does have a significant adverse effect on the heat deflection temperature of the produced impact modified with a polycarbonate / PBT blend using a high PBT content. This is believed to be due to incorporation of the impurities as co-monomers in the PBT backbone resulting in a decrease in the crystallinity of the polymer. Surprisingly, even low impurity levels result in significant changes to the heat deflection temperature of the impact modified with a polycarbonate / PBT blend using a high PBT content. If the total impurities present in the dimethyl terephthalate are more than only 4.2 wt.%, the impact modified with a polycarbonate / PBT blend using a high PBT content may have a significantly different heat deflection temperature. To ensure that the impact modified with a polycarbonate / PBT blend using a high PBT content made from recycled dimethyl terephthalate has an equivalent heat deflection temperature, the recycled dimethyl terephthalate must contain less than 4.2 wt.% of impurities.

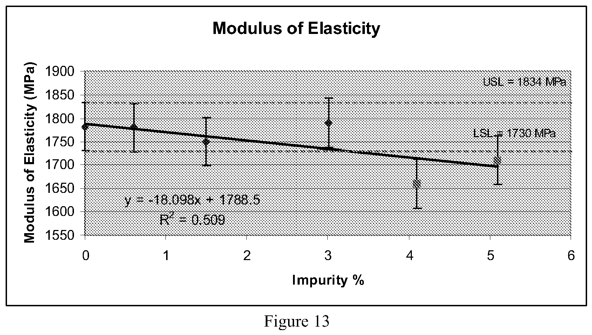

The modulus of elasticity measurements of the produced impact modified with a polycarbonate / PBT blend using a high PBT content are given in Figure 13. The modulus of elasticity measurements for Examples 31-34 show a significant reduction when impurities are present in the dimethyl terephthalate used to make the impact modified with a polycarbonate / PBT blend using a high PBT content. Surprisingly, the presence of the co-monomers in the amounts used in Examples 1-4 does have a significant adverse effect on the modulus of elasticity of the produced impact modified with a polycarbonate / PBT blend using a high PBT content. If the total impurities present in the dimethyl terephthalate are more than only 3.2 wt.%, the impact modified with a polycarbonate / PBT blend using a high PBT content may have a significantly different total energy measured by the biaxial impact test. To ensure that the impact modified with a polycarbonate / PBT blend using a high PBT content made from recycled dimethyl terephthalate has an equivalent modulus of elasticity, the recycled dimethyl terephthalate must contain less than 3.2 wt.% of impurities. To ensure that the impact modified with a polycarbonate / PBT blend using a high PBT content made from recycled dimethyl terephthalate has equivalent properties, the recycled dimethyl terephthalate must contain less than 3.2 wt.% of impurities.

## Claims

1. A flame retardant poly(butylene terephthalate) composition comprising
(a) a poly(butylene terephthalate) copolymer reaction product of 1,4-butanediol and a dimethyl terephthalate residual composition comprising a dimethyl terephthalate derived from a terephthalic-containing polyester homopolymer, terephthalic-containing polyester copolymer, or a combination thereof; and from more than 0 to less than 3.6 wt.% of a residual component selected from dimethyl isophthalate, cyclohexane dimethanol, diethylene glycol, triethylene glycol, and combinations thereof; and
(b) a flame retarding amount of a brominated polycarbonate,
wherein the flame retardant composition has a heat deflection temperature ranging from 61 °C to 74°C when measured at 1.82 MPa in accordance with ASTM Method D648.

2. The flame retardant poly(butylene terephthalate) composition of Claim 1,
wherein
the dimethyl terephthalate residual composition comprises more than 0 to less than or equal to 0.9 weight % of the residual component; and
the flame retardant composition has a heat deflection temperature ranging from 150°C to 156°C when measured at 0.455 MPa in accordance with ASTM Method D648.

3. A flame retardant poly(butylene terephthalate) composition comprising
from 30 to 90 wt.% of the poly(butylene terephthalate) copolymer reaction product of 1,4-butane diol and the dimethyl terephthalate residual composition comprising from more than 0 to less than 3.6 wt.% of a residual component selected from dimethyl isophthalate, cyclohexane dimethanol, diethylene glycol, triethylene glycol, and combinations thereof of Claim 1;
from 10 to 80 wt.% of a flame retardant brominated polycarbonate;
from 10 to 80 wt.% of a polycarbonate;
from more than 0 to 20 wt.% of an additional flame retardant;
from more than 0 to 2 wt.% of an antioxidant;
from more than 0 to 2 wt.% of a mold release agent;
from more than 0 to 2 wt.% of an anti-drip agent; and
from more than 0 to 2 wt.% of a quencher.

4. The flame retardant poly(butylene terephthalate) composition of any of Claims 1-3, wherein the brominated polycarbonate is a copolymer derived from bisphenol A and tetrabromobisphenol A.

5. The flame retardant poly(butylene terephthalate) composition of any of Claims 1-4, wherein the brominated polycarbonate comprises a combination of a non-brominated polycarbonate and a polycarbonate comprising brominated units.

6. The flame retardant poly(butylene terephthalate) composition of any of Claims 1-5, further comprising an additional flame retardant selected from sulfonate salts, brominated non-polycarbonate compounds, antimony flame retardants, phosphorus-containing flame retardants, nitrogen-containing flame retardants, phosphinate salts, diphosphinate salts, and combinations thereof.

7. The flame retardant poly(butylene terephthalate) composition of any of Claims 1-6, further comprising an additive selected from charring polymers, antidrip agents, antioxidants, thermal stabilizers, radiation stabilizers, and ultraviolet light absorbing additives, mold release agents, plasticizers, quenchers, lubricants, antistatic agents, dyes, pigments, laser marking additives, processing aids, and combinations thereof.

8. The flame retardant poly(butylene terephthalate) composition of any of Claims 4-6, further comprising an antioxidant, a mold release, an anti-drip agent, and a quencher.

9. The flame retardant poly(butylene terephthalate) composition of any of Claims 4-6, further comprising a polycarbonate, an additional flame retardant, and anti-drip agent.

10. The flame retardant poly(butylene terephthalate) composition of any of Claims 3-9, wherein the polycarbonate is a bisphenol A polycarbonate.

11. A method of manufacture of the flame retardant poly(butylene terephthalate) composition of any of Claims 1-10, comprising melt blending the components.

12. An article comprising the flame retardant poly(butylene terephthalate) composition of Claim 1.

13. The article of Claim 12, wherein the article is in the form of a housing having at least one surface comprising the composition of any of Claims 4-9.

14. The article of Claim 13, wherein the housing is a housing of an electrical connector.

15. A method of manufacture of an article, comprising molding, extruding, shaping, injection molding, or calendering the composition of any of Claims 1-10.

## Patentansprüche

1. Flammhemmende Poly(butylenterephthalat)-Zusammensetzung, umfassend:
(a) ein Poly(butylenterephthalat)-Copolymer als Reaktionsprodukt von 1,4-Butandiol und einer Dimethylterephthalat-Rest-Zusammensetzung, umfassend ein Dimethylterephthalat, abgeleitet von einem terephthalsäurehaltigen Polyester-Homopolymer, terephthalsäurehaltigen Polyester-Copolymer, oder einer Kombination davon; und von mehr als 0 Gew.-% bis weniger als 3,6 Gew.-% einer Restkomponente, ausgewählt aus Dimethylisophthalat, Cyclohexandimethanol, Diethylenglykol, Triethylenglykol, und Kombinationen davon; und
(b) eine flammhemmende Menge eines bromierten Polycarbonats,
wobei die flammhemmende Zusammensetzung eine von 61 °C bis 74 °C reichende Wärmeformbeständigkeitstemperatur bei Messung bei 1,82 MPa gemäß der Methode ASTM D 648 aufweist.

2. Flammhemmende Poly(butylenterephthalat)-Zusammensetzung nach Anspruch 1, wobei die Dimethylterephthalat-Rest-Zusammensetzung mehr als 0 Gew.-% bis weniger als oder gleich 0,9 Gew.-% der Restkomponente umfasst; und
die flammhemmende Zusammensetzung eine von 150 °C bis 156 °C reichende Wärmeformbeständigkeitstemperatur bei Messung bei 0,455 MPa gemäß der Methode ASTM D 648 aufweist.

3. Flammhemmende Poly(butylenterephthalat)-Zusammensetzung, umfassend:
von 30 Gew.-% bis 90 Gew.-% des Poly(butylenterephthalat)-Copolymers als Reaktionsprodukt von 1,4-Butandiol und der Dimethylterephthalat-Rest-Zusammensetzung, umfassend von mehr als 0 Gew.-% bis weniger als 3,6 Gew.-% einer Restkomponente, ausgewählt aus Dimethylisophthalat, Cyclohexandimethanol, Diethylenglykol, Triethylenglykol, und Kombinationen davon, nach Anspruch 1;
von 10 Gew.-% bis 80 Gew.-% eines flammhemmenden bromierten Polycarbonats;
von 10 Gew.-% bis 80 Gew.-% eines Polycarbonats;
von mehr als 0 Gew.-% bis 20 Gew.-% eines zusätzlichen Flammhemmers;
von mehr als 0 Gew.-% bis 2 Gew.-% eines Antioxidans;
von mehr als 0 Gew.-% bis 2 Gew.-% eines Entformungsmittels;
von mehr als 0 Gew.-% bis 2 Gew.-% eines Antitropfmittels; und
von mehr als 0 Gew.-% bis 2 Gew.-% eines Quenchers.

4. Flammhemmende Poly(butylenterephthalat)-Zusammensetzung nach einem der Ansprüche 1-3, wobei das bromierte Polycarbonat ein von Bisphenol A und Tetrabrombisphenol A abgeleitetes Copolymer ist.

5. Flammhemmende Poly(butylenterephthalat)-Zusammensetzung nach einem der Ansprüche 1-4, wobei das bromierte Polycarbonat eine Kombination aus einem nicht bromierten Polycarbonat und einem Polycarbonat mit bromierten Einheiten umfasst.

6. Flammhemmende Poly(butylenterephthalat)-Zusammensetzung nach einem der Ansprüche 1-5, weiterhin umfassend einen zusätzlichen Flammhemmer, ausgewählt aus Sulfonatsalzen, bromierten Nichtpolycarbonatverbindungen, Antimon-Flammhemmern, phosphorhaltigen Flammhemmern, stickstoffhaltigen Flammhemmern, Phosphinatsalzen, Diphosphinatsalzen, und Kombinationen davon.

7. Flammhemmende Poly(butylenterephthalat)-Zusammensetzung nach einem der Ansprüche 1-6, weiterhin umfassend ein Additiv, ausgewählt aus verkohlenden Polymeren, Antitropfmitteln, Antioxidantien, Wärmestabilisatoren, Strahlungsstabilisatoren und UV-Licht absorbierenden Additiven, Entformungsmitteln, Weichmachern, Quenchern, Schmiermitteln, Antistatika, Farbstoffen, Pigmenten, Additiven zur Lasermarkierung, Verarbeitungshilfsmitteln und Kombinationen davon.

8. Flammhemmende Poly(butylenterephthalat)-Zusammensetzung nach einem der Ansprüche 4-6, weiterhin umfassend ein Antioxidans, ein Entformungsmittel, ein Antitropfmittel und einen Quencher.

9. Flammhemmende Poly(butylenterephthalat)-Zusammensetzung nach einem der Ansprüche 4-6, weiterhin umfassend ein Polycarbonat, einen zusätzlichen Flammhemmer und ein Antitropfmittel.

10. Flammhemmende Poly(butylenterephthalat)-Zusammensetzung nach einem der Ansprüche 3-9, wobei das Polycarbonat ein Bisphenol-A-Polycarbonat ist.

11. Verfahren zur Herstellung der flammhemmenden Poly(butylenterephthalat)-Zusammensetzung nach einem der Ansprüche 1-10, umfassend Schmelzmischen der Komponenten.

12. Artikel, umfassend die flammhemmende Poly(butylenterephthalat)-Zusammensetzung nach Anspruch 1.

13. Artikel nach Anspruch 12, wobei der Artikel in Form eines Gehäuses mit mindestens einer Fläche, umfassend die Zusammensetzung nach einem der Ansprüche 4-9, vorliegt.

14. Artikel nach Anspruch 13, wobei das Gehäuse ein Gehäuse eines elektrischen Steckverbinders ist.

15. Verfahren zur Herstellung eines Artikels, umfassend Formpressen, Extrudieren, Formen, Spritzgießen oder Kalandrieren der Zusammensetzung nach einem der Ansprüche 1-10.

## Revendications

1. Composition de poly(butylène téréphtalate) retardatrice de flamme, comprenant
(a) un produit réactionnel de copolymère de poly(butylène téréphtalate) du 1,4-butanediol et d'une composition résiduelle de diméthyl téréphtalate comprenant un diméthyl téréphtalate dérivé d'un homopolymère de polyester contenant un acide téréphtalique, d'un copolymère de polyester contenant un acide téréphtalique, ou d'une combinaison de ceux-ci ; et plus de 0 à moins de 3,6 % en poids d'un composant résiduel choisi parmi le diméthyl isophtalate, le cyclohexane diméthanol, le diéthylène glycol, le triéthylène glycol, et des combinaisons de ceux-ci ; et
(b) une quantité retardatrice de flamme d'un polycarbonate bromé,
dans laquelle la composition retardatrice de flamme possède une température de distorsion à la chaleur allant de 61°C à 74°C quand elle est mesurée à 1,82 MPa d'après la méthode ASTM D648.

2. Composition de poly(butylène téréphtalate) retardatrice de flamme selon la revendication 1, dans laquelle la composition résiduelle de diméthyl téréphtalate comprend plus de 0 à moins ou autant que 0,9 % en poids du composant résiduel ; et
la composition retardatrice de flamme possède une température de distorsion à la chaleur allant de 150°C à 156°C quand elle est mesurée à 0,455 MPa d'après la méthode ASTM D648.

3. Composition de poly(butylène téréphtalate) retardatrice de flamme, comprenant
de 30 à 90 % en poids du produit réactionnel de copolymère de polybutylène téréphtalate du 1,4-butanediol et de la composition résiduelle de diméthyl téréphtalate comprenant plus de 0 à moins de 3,6 % en poids d'un composant résiduel choisi parmi le diméthyl isophtalate, le cyclohexane diméthanol, le diéthylène glycol, le triéthylène glycol, et des combinaisons de ceux-ci selon la revendication 1 ;
de 10 à 80 % en poids d'un polycarbonate bromé retardateur de flamme ;
de 10 à 80 % en poids d'un polycarbonate ;
de plus de 0 à 20 % en poids d'un retardateur de flamme additionnel ;
de plus de 0 à 2 % en poids d'un antioxydant ;
de plus de 0 à 2 % en poids d'un agent de démoulage ;
de plus de 0 à 2 % en poids d'un agent anti-goutte ; et
de plus de 0 à 2 % en poids d'un agent d'extinction.

4. Composition de poly(butylène téréphtalate) retardatrice de flamme selon l'une quelconque des revendications 1 à 3, dans laquelle le polycarbonate bromé est un copolymère dérivé du bisphénol A et du tétrabromobisphénol A.

5. Composition de poly(butylène téréphtalate) retardatrice de flamme selon l'une quelconque des revendications 1 à 4, dans laquelle le polycarbonate bromé comprend une combinaison d'un polycarbonate non bromé et d'un polycarbonate comprenant des motifs bromés.

6. Composition de poly(butylène téréphtalate) retardatrice de flamme selon l'une quelconque des revendications 1 à 5, comprenant en outre un retardateur de flamme additionnel choisi parmi les sels de sulfonate, les composés non polycarbonate bromés, les retardateurs de flamme à l'antimoine, les retardateurs de flamme contenant du phosphore, des retardateurs de flamme contenant de l'azote, les sels de phosphinate, les sels de diphosphinate, et des combinaisons de ceux-ci.

7. Composition de poly(butylène téréphtalate) retardatrice de flamme selon l'une quelconque des revendications 1 à 6, comprenant en outre un additif choisi parmi les polymères de carbonisation, les agents anti-goutte, les antioxydants, les stabilisateurs thermiques, les stabilisateurs de rayonnement, et les additifs absorbant la lumière ultraviolette, les agents de démoulage, les plastifiants, les agents d'extinction, les lubrifiants, les agents antistatiques, les colorants, les pigments, les additifs de marquage laser, les auxiliaires de transformation et des combinaisons de ceux-ci.

8. Composition de poly(butylène téréphtalate) retardatrice de flamme selon l'une quelconque des revendications 4 à 6, comprenant en outre un antioxydant, un agent de démoulage, un agent anti-goutte et un agent d'extinction.

9. Composition de poly(butylène téréphtalate) retardatrice de flamme selon l'une quelconque des revendications 4 à 6, comprenant en outre un polycarbonate, un retardateur de flamme additionnel, et un agent anti-goutte.

10. Composition de poly(butylène téréphtalate) retardatrice de flamme selon l'une quelconque des revendications 3 à 9, dans laquelle le polycarbonate est un polycarbonate de bisphénol A.

11. Méthode de fabrication de la composition de poly(butylène téréphtalate) retardatrice de flamme selon l'une quelconque des revendications 1 à 10, comprenant le mélange à l'état fondu des composants.

12. Article comprenant la composition de poly(butylène téréphtalate) retardatrice de flamme selon la revendication 1.

13. Article selon la revendication 12, dans lequel l'article se trouve sous la forme d'un boîtier ayant au moins une surface comprenant la composition selon l'une quelconque des revendications 4 à 9.

14. Article selon la revendication 12, dans lequel le boîtier est un boîtier d'un connecteur électrique.

15. Méthode de fabrication d'un article, comprenant le moulage, l'extrusion, le formage, le moulage par injection ou le calandrage de la composition selon l'une quelconque des revendications 1 à 10.
